(19) Europäisches Patentamt
European Patent Office
Office européen des brevets

(11) Veröffentlichungsnummer: **0 453 846 A2**

(12) # EUROPÄISCHE PATENTANMELDUNG

(21) Anmeldenummer: **91105486.4**

(22) Anmeldetag: **06.04.91**

(51) Int. Cl.[5]: **C07D 263/38**, C07D 271/10, C07D 277/34, C07D 285/08, C07D 285/12, C07D 249/12, C07D 417/04, A01N 43/76, A01N 43/78, A01N 43/653, A01N 43/82

(30) Priorität: **21.04.90 DE 4012793**
**03.10.90 DE 4031158**

(43) Veröffentlichungstag der Anmeldung:
**30.10.91 Patentblatt 91/44**

(84) Benannte Vertragsstaaten:
**BE CH DE DK ES FR GB IT LI NL**

(71) Anmelder: **BAYER AG**

**W-5090 Leverkusen 1 Bayerwerk(DE)**

(72) Erfinder: **Haas, Wilhelm, Dr.**
**Nordstrasse 1**
**W-5014 Kerpen 3(DE)**
Erfinder: **Förster, Heinz, Dr.**
**Am Eckbusch 47**
**W-5600 Wuppertal 1(DE)**
Erfinder: **Kirsten, Rolf, Dr.**
**Carl-Langhans-Strasse 27**
**W-4019 Monheim(DE)**
Erfinder: **Drewes, Mark Wilhelm, Dr.**
**Grünstrasse 30**
**W-4018 Langenfeld(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Aryloxycarbonylverbindungen (11.11.11).**

(57) Beschrieben werden neue Aryloxycarbonylverbindungen der Formel (I)

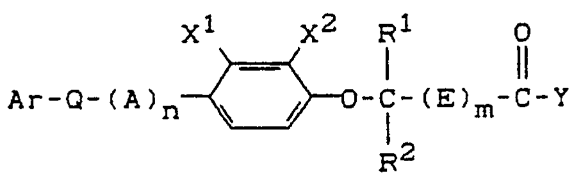

(I)

in welcher m, n, Ar, Q, A, $X^1$, $X^2$, $R^1$, $R^2$, E und Y die in der Beschreibung angegebene Bedeutung haben sowie mehrere Verfahren und neue Zwischenprodukte zu ihrer Herstellung.

Verwendet werden die neuen Verbindungen der Formel (I) als Herbizide.

EP 0 453 846 A2

Die Erfindung betrifft neue Aryloxycarbonylverbindungen, mehrere Verfahren und neue Zwischenprodukte zu ihrer Herstellung sowie ihre Verwendung als Herbizide.

Es ist bereits bekannt geworden, daß Phenoxypropionsäure-Derivate herbizide Eigenschaften besitzen (vgl. DE-OS 22 23 894). So kann zum Beispiel der 2-[4-(2,4-Dichlorphenoxy)-phenoxy]-propionsäuremethylester zur Unkrautbekämpfung eingesetzt werden. Die Wirkung dieses Stoffes ist jedoch insbesondere beim Einsatz niedriger Aufwandmengen nicht immer ausreichend.

Es wurden nun neue Aryloxycarbonylverbindungen der allgemeinen Formel (I)

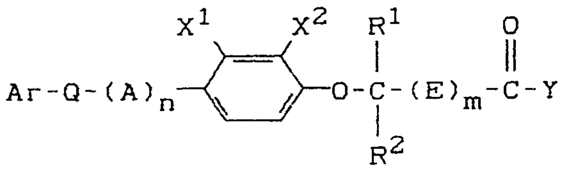 (I)

in welcher

| | |
|---|---|
| m und n | für die Zahlen 0 oder 1 stehen, |
| Ar | für Aryl oder Heteroaryl steht, |
| Q | für Heteroarylen steht, |
| A | für Sauerstoff, Schwefel oder Methylen steht, |
| $X^1$ und $X^2$ | gleich oder verschieden sind und für Wasserstoff, Halogen, Alkyl, Halogenalkyl, Alkoxy oder Halogenalkoxy stehen, |
| $R^1$ und $R^2$ | gleich oder verschieden sind und für Wasserstoff, Alkyl oder Halogenalkyl stehen, |
| E | für eine gegebenenfalls substituierte Ethylen- oder Vinylen-Gruppe steht, und |
| Y | für den Rest einer Carbonsäure oder eines Carbonsäurederivats steht |

gefunden.

Die Verbindungen der Formel (I) enthalten gegebenenfalls ein asymmetrisch substituiertes Kohlenstoffatom und können in diesem Fall in verschiedenen enantiomeren Formen vorliegen. Die Erfindung betrifft sowohl die möglichen einzelnen Isomeren als auch Gemische dieser Isomeren.

Weiter wurde gefunden, daß man die neuen Aryloxycarbonylverbindungen der Formel (I) erhält, wenn man

(a) Arylverbindungen der allgemeinen Formel (II)

$Ar\text{-}Q\text{-}X^3$ (II)

in welcher

| | |
|---|---|
| Ar und Q | die oben angegebenen Bedeutungen haben und |
| $X^3$ | für eine nucleofuge Gruppe steht, |

mit Hydroxy- oder Mercaptoaryloxycarbonylverbindungen der allgemeinen Formel (III)

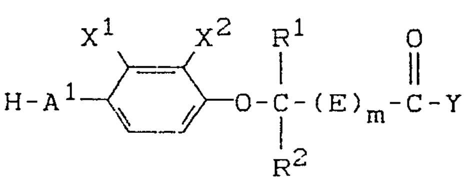 (III)

in welcher

m, $X^1$, $X^2$, $R^1$, $R^2$, E und Y die oben angegebenen Bedeutungen haben und

$A^1$ für Sauerstoff oder Schwefel steht,

gegebenenfalls in Gegenwart eines Säureakzeptors und gegebenenfalls in Gegenwart eines Verdünnungsmittels umsetzt, oder wenn man

(b) Phenolderivate der allgemeinen Formel (IV)

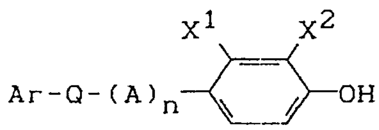

(IV)

in welcher

n, Ar, Q, A, $X^1$ und $X^2$ die oben angegebenen Bedeutungen haben,

mit Carbonsäurederivaten der allgemeinen Formel (V)

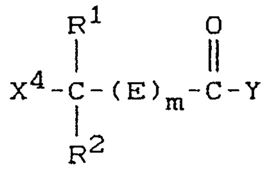

(V)

in welcher

m, $R^1$, $R^2$, E und Y die oben angegebenen Bedeutungen haben und

$X^4$ für eine nucleofuge Gruppe steht,

gegebenenfalls in Gegenwart eines Säureakzeptors und gegebenenfalls in Gegenwart eines Verdünnungsmittels umsetzt.

Schließlich wurde gefunden, daß die neuen Aryloxycarbonylverbindungen der allgemeinen Formel (I) interessante herbizide Eigenschaften aufweisen.

Überraschenderweise zeigen die erfindungsgemäßen Aryloxycarbonylverbindungen der Formel (I) bei guter Verträglichkeit gegenüber wichtigen Kulturpflanzen wesentlich stärkere Wirkung gegen bestimmte Problemunkräuter, wie insbesondere Hirsearten, als die bekannte Verbindung 2-[4-(2,4-Dichlorphenoxy)-phenoxy]-propionsäuremethylester, welcher ein strukturell ähnlicher vorbekannter Wirkstoff gleicher Wirkungsrichtung ist.

Bevorzugte Substituenten bzw. Bereiche der in den oben und nachstehend erwähnten Formeln aufgeführten Reste werden im folgenden erläutert:

Alkyl steht einzeln oder in zusammengesetzten Resten für geradkettiges oder verzweigtes Alkyl mit 1 bis 6 und vorzugsweise mit 1 bis 4 Kohlenstoffatomen; beispielhaft und vorzugsweise seien genannt Methyl, Ethyl, n-und i-Propyl, n-, i-, s- und t-Butyl.

Alkoxy steht in den allgemeinen Formeln für geradkettiges oder verzweigtes Alkoxy mit 1 bis 6 und bevorzugt 1 bis 4 Kohlenstoffatomen; beispielhaft und vorzugsweise seien genannt: Methoxy, Ethoxy, n- und i-Propoxy, n-, i-, s- und t-Butoxy.

Halogenalkyl und Halogenalkoxy stehen in den allgemeinen Formeln für geradkettiges oder verzweigtes Halogenalkyl bzw. Halogenalkoxy mit 1 bis 4 und bevorzugt mit 1 oder 2 Kohlenstoffatomen und jeweils 1 bis 9 und bevorzugt 1 bis 5, gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluor-methyl, Trifluorchlorethyl, Chlorbutyl und Fluorbutyl sowie die diesen entsprechenden Halogenalkoxyreste.

Aryl in den allgemeinen Formeln steht für gegebenenfalls substituiertes Aryl mit vorzugsweise 6 bis 10 Kohlenstoffatomen im Arylteil. Beispielhaft und vorzugsweise seien unsubstituiertes oder substituiertes Phenyl oder Naphthyl, insbesondere Phenyl genannt.

Heteroaryl in den allgemeinen Formeln steht für einen gegebenenfalls substituierten 5- bis 6-gliedrigen Ring, der 1 bis 3, bevorzugt 1 oder 2 gleiche oder verschiedene Heteroatome enthält. Als Heteroatome seien vorzugsweise Sauerstoff, Schwefel und Stickstoff genannt; beispielhaft und vorzugsweise seien genannt: Pyrimidinyl, Pyrrolyl, Imidazol, Isothiazolyl, Oxazolyl, Thienyl, Furyl, Pyridazinyl, Pyrazinyl, Isoxazolyl, Thiazolyl, Pyrazolyl und insbesondere Pyridyl.

Heteroarylen in den allgemeinen Formeln steht für einen gegebenenfalls durch $C_1$-$C_4$-Alkyl substituierten, zweifach verknüpften 5- oder 6-gliedrigen Ring, der 1 bis 4, bevorzugt 1 bis 3 gleiche oder verschiedene Heteroatome enthält, Als Heteroatome seien vorzugsweise Sauerstoff, Schwefel oder Stickstoff genannt; beispielhaft und vorzugsweise seien Pyrazin-2,6-diyl, Pyrazin-2,5-diyl, 1,2,4-Triazin-3,5-diyl, 1,2,4-Triazin-3,6-diyl, 1,3-Imidazol-2,5-diyl, 1,3-Imidazol-2,4-diyl oder 1,2,4-Triazol-3,5-diyl (welche je-weils

gegebenenfalls durch $C_1$-$C_4$-Alkyl substituiert sind), 1,3-Oxazol-2,5-diyl, 1,3-Thiazol-2,5-diyl, 1,3-Oxazol-2,4-diyl oder 1,3-Thiazol-2,4-diylund insbesondere 1,3,4-Oxadiazol-2,5-diyl, 1,3,4-Thiadiazol-2,5-diyl, 1,2,4-Oxadiazol-3,5-diyl und 1,2,4-Thiadiazol-3,5-diyl, genannt.

Der Rest einer Carbonsäure oder eines Carbonsäurederivats in der Definition von Y steht vorzugsweise für

Halogen, Hydroxy, Amino, Hydrazino, Cyano, Cyanamino, Hydroxyamino, Isoxazolidinyl oder für jeweils gegebenenfalls substituiertes Alkylamino, Alkenylamino, Alkinylamino, Arylamino, Aralkylamino, Alkoxycarbonylalkylamino, Dialkylamino, Pyrrolidino, Piperidino, Morpholino, Dialkenylamino, Alkylsulfonylamino, Arylsulfonylamino, Alkoxyamino, Alkylsulfonylhydrazino, Arylsulfonylhydrazino, Alkylthio, Arylthio, Aralkylthio, Alkoxycarbonylalkylthio oder für die Gruppierung -O-$R^3$ worin

$R^3$ für jeweils gegebenenfalls substituiertes Alkyl, Alkenyl, Alkinyl, Alkoxyalkyl, Alkoxyalkoxyalkyl, Alkylthioalkyl, Alkylsulfinylalkyl, Alkylsulfonylalkyl, Aryloxyalkyl, Trialkylsilylalkyl, Arylthioalkyl, Aralkoxyalkyl, Aralkylthioalkyl, Alkoxycarbonylalkyl, Alkylaminocarbonylalkyl, Aralkyl, Azolylalkyl, Alkylidenamino, oder für ein Ammonium-, Alkylammonium-, Alkalimetall- oder Erdalkalimetalläquivalent steht, oder

$R^3$ für die Gruppierung

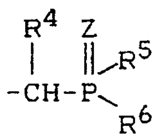

steht, worin

$R^4$ für Wasserstoff, Alkyl, Aryl, Furyl, Thienyl oder Pyridyl steht,

$R^5$ für Alkyl oder Alkoxy steht,

$R^6$ für Alkoxy steht und

Z für Sauerstoff oder Schwefel steht,

oder

$R^3$ für die Gruppierung $-(CH_2)_p-R^7$ steht, worin

R7 für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Isoxazolyl, Thiazolyl, Thiadiazolyl, Dioxolanyl, Perhydropyrrolyl, Oxoperhydropyrrolyl, Pyrazolyl, Pyridinyl oder Pyrimidinyl steht und

p für die Zahlen 0, 1 oder 2 steht.

Halogen steht im allgemeinen als Substituent oder in dem Rest Halogenalkyl für Fluor, Chlor, Brom und Iod, insbesondere für Fluor und Chlor.

Als Substituenten für die Ethylen- und Vinylen-Gruppe und für die Hetaryl- und Arylreste seien vorzugsweise genannt:

Halogen; geradkettiges oder verzweigtes Alkyl mit 1 bis 6, bevorzugt mit 1 bis 4 Kohlenstoffatomen und besonders bevorzugt Methyl und Ethyl (die beispielhafte Aufzählung entspricht der weiter oben gegebenen); geradkettiges oder verzweigtes Alkoxy und Akylthio mit jeweils 1 bis 6, bevorzugt mit 1 bis 4 Kohlenstoffatomen und besonders bevorzugt mit 1 oder 2 Kohlenstoffatomen (beispielhaft seien genannt: Methoxy, Ethoxy, n- und i-Propoxy, n-, i-, s- und t-Butoxy, Methylthio, Ethylthio, n- und i-Propylthio); geradkettiges oder verzweigtes Halogenalkyl, Halogenalkoxy und Halogenalkylthio mit vorzugsweise je 1 bis 4 Kohlenstoffatomen, besonders bevorzugt mit jeweils 1 oder 2 Kohlenstoffatomen und jeweils 1 bis 9, vorzugsweise 1 bis 5, gleichen oder verschiedenen Halogenatomen wie unter Halogen definiert (beispielhaft seien genannt: Fluormethyl, Chlormethyl, Brommethyl, Fluorethyl, Chlorethyl, Brommethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy, Trifluorchlorethoxy und Trifluormethylthio); geradkettiges oder verzweigtes Alkylsulfinyl und Alkylsulfonyl mit je 1 bis 6 und vorzugsweise 1 bis 4 Kohlenstoffatomen im Alkylteil, besonders bevorzugt mit 1 oder 2 Kohlenstoffatomen im Alkylteil; Hydroxy, Nitro und Cyano.

Die hier aufgeführten Definitionen gelten in entsprechender Weise auch für die im folgenden aufgeführten bevorzugten Kombinationen von Resten.

Die Erfindung betrifft vorzugsweise Verbindungen der Formel (I),
in welcher

m und n für die Zahlen 0 oder 1 stehen,

Ar für Phenyl oder Pyridyl steht, welche jeweils gegebenenfalls durch Halogen, Cyano, Nitro und/oder durch jeweils gegebenenfalls halogen-substituiertes Alkyl, Alkoxy, Alkylthio, Alkylsulfinyl oder Alkylsulfonyl mit jeweils 1 bis 6 Kohlenstoffatomen im Alkylteil substituiert sind,

Q für einen fünf- oder sechsgliedrigen Heteroarylenrest steht, welcher 2 oder 3 Stickstoffatome (von denen gegebenenfalls eines alkyiert ist) oder 1 oder 2 Stickstoffatome und zusätzlich 1 Sauerstoffatom oder Schwefelatom als Heteroatome enthält, steht,

A für Sauerstoff, Schwefel oder Methylen steht,

$X^1$ und $X^2$ gleich oder verschieden sind und für Wasserstoff, Halogen, Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen stehen,

$R^1$ und $R^2$ gleich oder verschieden sind und für Wasserstoff, Alkyl oder Halogenalkyl mit jeweils 1 bis 4 Kohlenstoffatomen und 1 bis 9 gleichen oder verschiedenen Halogenatomen stehen,

E für die Gruppierungen -CH = CH- oder

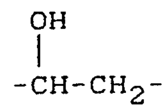

steht und

Y für Halogen insbesondere Chlor, Hydroxy, Amino, Hydrazino, Cyano, Cyanamino, Hydroxyamino, Isoxazolidinyl oder für jeweils gegebenenfalls durch Halogen substituiertes $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Di-($C_1$-$C_4$-alkyl)-amino, Pyrrolidino, Piperidino, Morpholino, Di-($C_3$-$C_4$-alkenyl)-amino, $C_1$-$C_4$-Alkylsulfonylamino, Phenylsulfonylamino, Tolylsulfonylamino, $C_1$-$C_6$-Alkoxyamino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^3$ steht, worin

$R^3$ für jeweils gegebenenfalls durch Fluor und/oder Chlor substituiertes $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxycarbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino, oder für ein Ammonium-, ein $C_1$-$C_4$-Alkylammonium-, ein Natrium-, Kalium- oder Calciumäquivalent steht, oder

$R^3$ für die Gruppierung

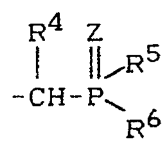

steht, worin

$R^4$ für Wasserstoff, $C_1$-$C_4$-Alkyl, Phenyl, Furyl, Thienyl oder Pyridyl steht,

$R^5$ für $C_1$-$C_4$-Alkyl oder $C_1$-$C_4$-Alkoxy steht,

$R^6$ für $C_1$-$C_4$-Alkoxy steht und

Z für Sauerstoff oder Schwefel steht,

oder

$R^3$ für die Gruppierung -$(CH_2)_p$-$R^7$ steht, worin

$R^7$ für einen gegebenenfalls durch Fluor, Chlor, Brom und/oder $C_1$-$C_4$-Alkyl substituierten heterocycli-

schen Rest aus der Reihe Furyl, Tetrahydrofuryl, Oxotetrahydrofuryl, Thienyl, Tetrahydrothienyl, Perhydropyranyl, Oxazolyl, Isooxazolyl, Thiazolyl, Thiadiazolyl, Dioxolanyl, Perhydropyrrolyl, Oxoperhydropyrrolyl, Pyridinyl, Pyrazolyl oder Pyrimidinyl steht und

p für die Zahlen 0, 1 oder 2 steht.

Besonders bevorzugt sind Verbindungen der Formel (I), in welcher

m und n für die Zahlen 0 oder 1 stehen,

Ar für Phenyl oder Pyridyl steht, welche jeweils gegebenenfalls durch Fluor, Chlor, Brom, Cyano, Nitro und/oder durch jeweils gegebenenfalls durch Fluor und/oder Chlor substituiertes $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert sind,

Q für Pyrazin-2,6-diyl, Pyrazin-2,5-diyl, 1,2,4-Triazin-3,5-diyl, 1,2,4-Triazin-3,6-diyl, 1,3-Imidazol-2,5-diyl, 1,3-Imidazol-2,4-diyl oder 1,2,4-Triazol-3,5-diyl (welche jeweils gegebenenfalls durch $C_1$-$C_4$-Alkyl substituiert sind), für 1,3,4-Oxadiazol-2,5-diyl, 1,3,4-Thiadiazol-2,5-diyl, 1,2,4-Oxadiazol-3,5-diyl, 1,2,4-Thiadiazol-3,5-diyl, 1,3-Oxazol-2,5-diyl, 1,3-Thiazol-2,5-diyl, 1,3-Oxazol-2,4-diyl oder 1,3-Thiazol-2,4-diyl steht,

A für Sauerstoff, Schwefel oder Methylen steht,

$X^1$ und $X^2$ gleich oder verschieden sind und für Wasserstoff, Fluor, Chlor oder Halogenalkyl mit 1 oder 2 Kohlenstoffatomen und 1 bis 5 gleichen oder verschiedenen Halogenatomen stehen,

$R^1$ und $R^2$ gleich oder verschieden sind und für Wasserstoff, $C_1$-$C_4$-Alkyl oder Halogenalkyl mit 1 bis 4 Kohlenstoffatomen und 1 bis 9 gleichen oder verschiedenen Halogenatomen stehen,

E für die Gruppierungen -CH = CH- oder

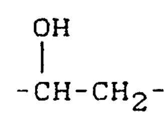

steht und

Y für Chlor, Hydroxy, Amino, Hydrazino, Cyano, Cyanamino, Hydroxyamino, Isoxazolidinyl oder für jeweils gegebenenfalls durch Fluor und/oder Chlor substituiertes $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Di-($C_1$-$C_4$-alkyl)-amino, Pyrrolidino, Piperidino, Morpholino, Di-($C_3$-$C_4$-alkenyl)-amino, $C_1$-$C_4$-Alkylsulfonylamino, Phenylsulfonylamino, Tolylsulfonylamino, $C_1$-$C_6$-Alkoxyamino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^3$ steht, worin

$R^3$ für jeweils gegebenenfalls durch Fluor und/oder Chlor substituiertes $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkoxy-$C_1$-$C_3$-alkoxy-$C_1$-$C_3$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxycarbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino, oder für ein Ammonium-, ein $C_1$-$C_4$-Alkylammonium-, ein Natrium-, Kalium- oder Calciumäquivalent steht, oder

$R^3$ für die Gruppierung

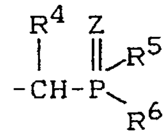

steht, worin

$R^4$ für Wasserstoff, $C_1$-$C_4$-Alkyl, Phenyl, Furyl, Thienyl oder Pyridyl steht,

$R^5$ für $C_1$-$C_4$-Alkyl oder $C_1$-$C_4$-Alkoxy steht,

$R^6$ für $C_1$-$C_4$-Alkoxy steht und

Z für Sauerstoff oder Schwefel steht,

oder

$R^3$ für die Gruppierung $-(CH_2)_p-R^7$ steht, worin

$R^7$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Isoxazolyl, Thiazolyl, Thiadiazolyl, Dioxolanyl, Perhydropyrrolyl, Oxoperhydropyrrolyl, Pyridinyl, Pyrazolyl oder Pyrimidinyl steht und

p für die Zahlen 0, 1 oder 2 steht.

Die Erfindung betrifft insbesondere Verbindungen der Formel (I), in welcher

m für die Zahl 0 steht,

n für die Zahl 0 oder 1 steht,

Ar für Phenyl oder Pyridyl steht, welche gegebenenfalls einfach oder zweifach durch Fluor und/oder Chlor und/oder einfach durch Methyl, Trifluormethyl, Methoxy, Difluormethoxy, Trifluormethoxy, Methylthio oder Trifluormethylthio substituiert sind,

Q für 1,3,4-Oxadiazol-2,5-diyl, 1,3,4-Thiadiazol-2,5-diyl, 1,2,4-Oxadiazol-3,5-diyl, 1,2,4-Thiadiazol-3,5-diyl, 1,2,4-Triazol-3,5-diyl, 1-Methyl-1,2,4-triazol-3,5-diyl, 4-Methyl-1,2,4-triazol-3,5-diyl, 1,3-Oxazol-2,5-diyl, 1,3-Oxazol-2,4-diyl, 1,3-Thiazol-2,5-diyl oder 1,3-Thiazol-2,4-diyl steht,

A für Sauerstoff steht,

$X^1$ und $X^2$ für Wasserstoff stehen,

$R^1$ für Methyl oder Wasserstoff steht,

$R^2$ für Wasserstoff steht und

Y für Chlor, Hydroxy, Amino, Isoxazolidinyl oder für jeweils gegebenenfalls durch F- und/oder Cl-substituiertes $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkyl-sulfonylamino, Phenylsulfonylamino, $C_1$-$C_4$-Alkoxyamino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung $-O-R^3$ steht, worin

$R^3$ für jeweils gegebenenfalls durch Cl- und/oder F-substituiertes $C_1$-$C_4$-Alkyl, $C_1$-$C_2$-Alkoxy-$C_1$-$C_2$-alkyl, $C_1$-$C_2$-Alkoxy-$C_1$-$C_2$-alkoxy-methyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl, Propargyl oder für ein Ammonium-, $C_1$-$C_3$-Alkylammonium-, Natrium-, oder Kaliumäquivalent steht, oder

$R^3$ für die Gruppierung $-(CH_2)_p-R^7$ steht, worin

$R^7$ für einen heterocyclischen Rest aus der Reihe Tetrahydrofuryl, Isoxazolyl oder Pyrazolyl steht und

p für die Zahlen 1 oder 2 steht.

Die jeweils möglichen Isomeren mit R-Konfiguration am Kohlenstoffatom, welches sich in α-Stellung zur Carbonylgruppe befindet, aus dem Bereich der oben als insbesondere bevorzugt definierten Verbindungen der Formel (I), werden ganz besonders bevorzugt.

Beispiele für die Verbindungen der Formel (I) sind in der nachstehenden Tabelle 1 aufgeführt.

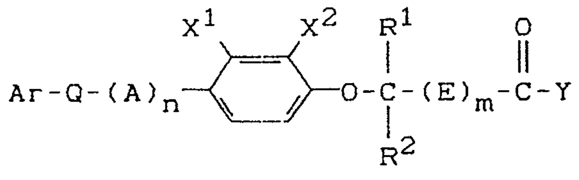

(I)

Tabelle 1: Beispiele für Verbindungen der Formel (I)

| Ar | Q | A | n | $X^1$ | $X^2$ | $R^1$ | $R^2$ | m | Y |
|---|---|---|---|---|---|---|---|---|---|
| 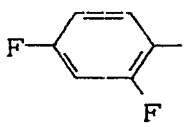 | 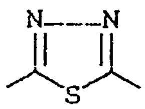 | O | 1 | H | H | $CH_3$ | H | 0 | OH |
| 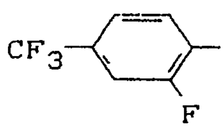 | 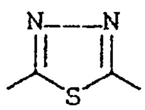 | O | 1 | H | H | $CH_3$ | H | 0 | $OCH_3$ |
| 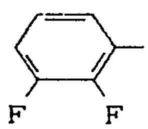 | 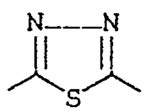 | O | 1 | H | H | $CH_3$ | H | 0 | $OC_2H_5$ |
| 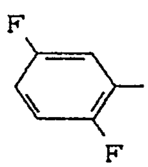 | 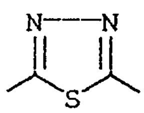 | O | 1 | H | H | $CH_3$ | H | 0 | OH |
| 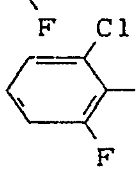 | 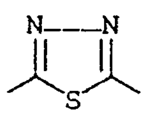 | O | 1 | H | H | $CH_3$ | H | 0 | $OCH_3$ |
| 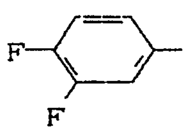 | 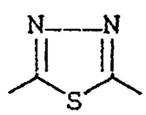 | O | 1 | H | H | $CH_3$ | H | 0 | $OC_2H_5$ |
| 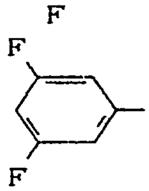 | 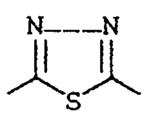 | O | 1 | H | H | $CH_3$ | H | 0 | OH |
| 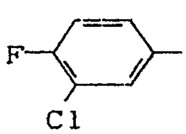 | 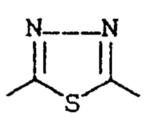 | O | 1 | H | H | $CH_3$ | H | 0 | $OCH_3$ |
| 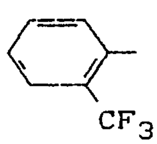 | 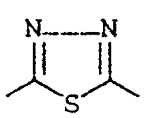 | O | 1 | H | H | $CH_3$ | H | 0 | $OC_2H_5$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 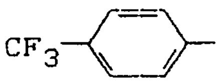 | 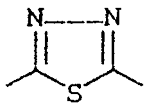 | O | 1 | H | H | $CH_3$ | H | 0 | OH |
| 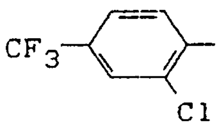 | 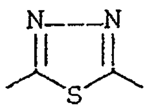 | O | 1 | H | H | $CH_3$ | H | 0 | $OCH_3$ |
| 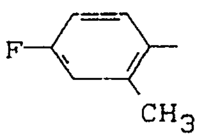 | 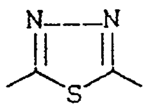 | O | 1 | H | H | $CH_3$ | H | 0 | $OC_2H_5$ |
| 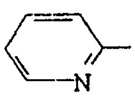 | 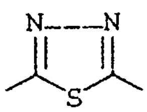 | O | 1 | H | H | $CH_3$ | H | 0 | OH |
| 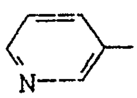 | 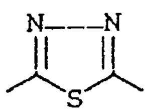 | O | 1 | H | H | $CH_3$ | H | 0 | $OCH_3$ |
| 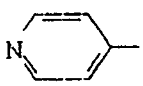 | 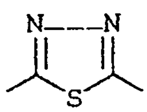 | O | 1 | H | H | $CH_3$ | H | 0 | $OC_2H_5$ |
| 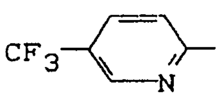 | 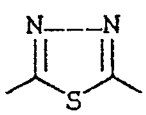 | O | 1 | H | H | $CH_3$ | H | 0 | OH |
| 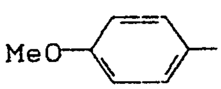 | 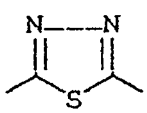 | O | 1 | H | H | $CH_3$ | H | 0 | $OCH_3$ |
| 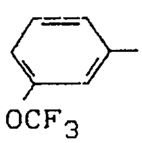 | 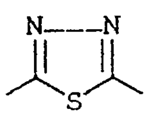 | O | 1 | H | H | $CH_3$ | H | 0 | $OC_2H_5$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 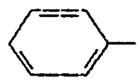 | 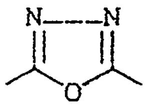 | O | 1 | H | H | $CH_3$ | H | 0 | OH |
| 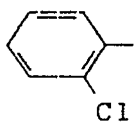 | 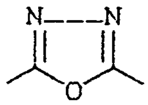 | O | 1 | H | H | $CH_3$ | H | 0 | $OCH_3$ |
| 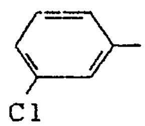 | 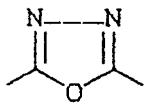 | O | 1 | H | H | $CH_3$ | H | 0 | $OCH_3$ |
| 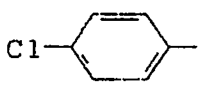 | 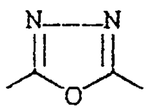 | O | 1 | H | H | $CH_3$ | H | 0 | $OC_2H_5$ |
| 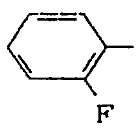 | 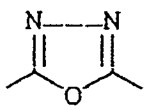 | O | 1 | H | H | $CH_3$ | H | 0 | OH |
| 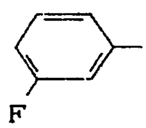 | 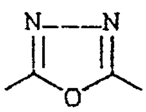 | O | 1 | H | H | $CH_3$ | H | 0 | $OC_2H_5$ |
| 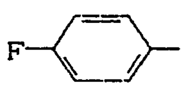 | 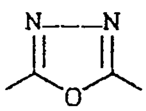 | O | 1 | H | H | $CH_3$ | H | 0 | $OC_2H_5$ |
| 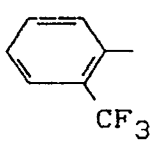 | 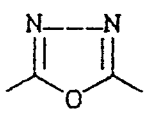 | O | 1 | H | H | $CH_3$ | H | 0 | $OC_2H_5$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 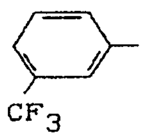 | 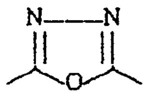 | O | 1 | H | H | $CH_3$ | H | O | OH |
| 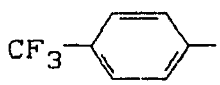 | 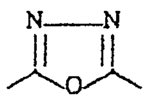 | O | 1 | H | H | $CH_3$ | H | O | $OCH_3$ |
| 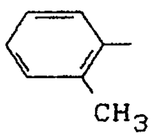 | 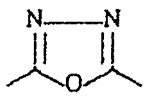 | O | 1 | H | H | $CH_3$ | H | O | $OCH_3$ |
| 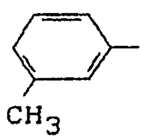 | 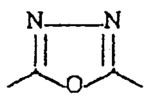 | O | 1 | H | H | $CH_3$ | H | O | $OC_2H_5$ |
| 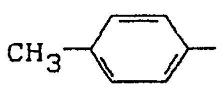 | 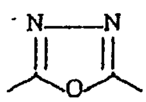 | O | 1 | H | H | $CH_3$ | H | O | OH |
| 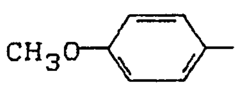 | 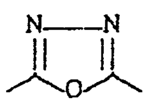 | O | 1 | H | H | $CH_3$ | H | O | $OC_2H_5$ |
| 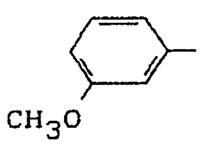 | 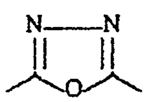 | O | 1 | H | H | $CH_3$ | H | O | $OC_2H_5$ |
| 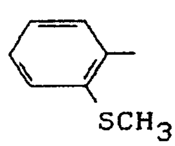 | 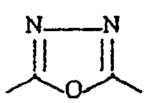 | O | 1 | H | H | $CH_3$ | H | O | $OC_2H_5$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 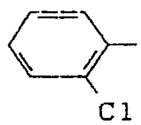 | 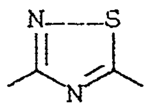 | O | 1 | H | H | $CH_3$ | H | 0 | $OCH_3$ |
| 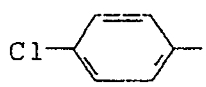 | 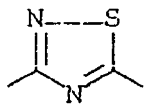 | O | 1 | H | H | $CH_3$ | H | 0 | $OCH_3$ |
| 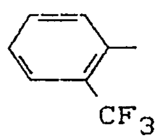 | 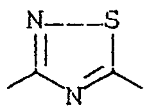 | O | 1 | H | H | $CH_3$ | H | 0 | $OC_2H_5$ |
| 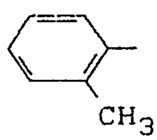 | 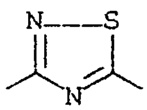 | O | 1 | H | H | $CH_3$ | H | 0 | $OC_3H_7$ |
| 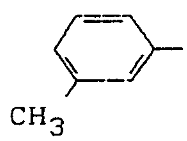 | 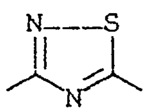 | O | 1 | H | H | $CH_3$ | H | 0 | OH |
| 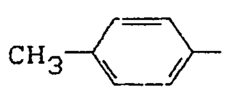 | 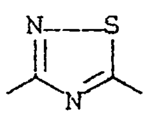 | O | 1 | H | H | $CH_3$ | H | 0 | $OCH_3$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 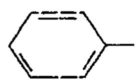 | 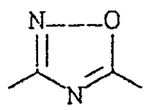 | O | 1 | H | H | $CH_3$ | H | 0 | $OC_2H_5$ |
| 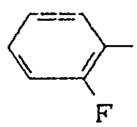 | 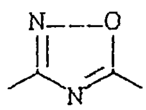 | O | 1 | H | H | $CH_3$ | H | 0 | $OC_2H_5$ |
| 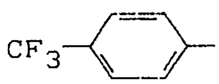 | 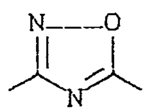 | O | 1 | H | H | $CH_3$ | H | 0 | $OC_2H_5$ |
| 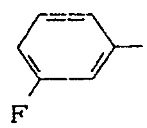 | 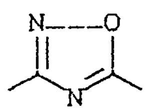 | O | 1 | H | H | $CH_3$ | H | 0 | $OC_2H_5$ |
| 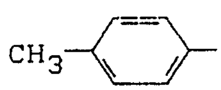 | 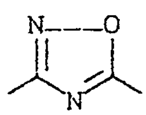 | O | 1 | H | H | $CH_3$ | H | 0 | OH |
| 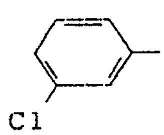 | 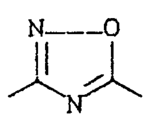 | O | 1 | H | H | $CH_3$ | H | 0 | $OC_2H_5$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 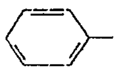 | 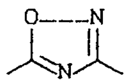 | O | 1 | H | H | $CH_3$ | H | 0 | OH |
| 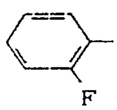 | 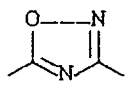 | O | 1 | H | H | $CH_3$ | H | 0 | OH |
| 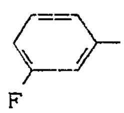 | 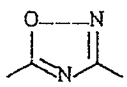 | O | 1 | H | H | $CH_3$ | H | 0 | OH |
| 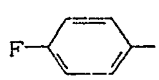 | 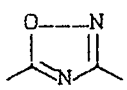 | O | 1 | H | H | $CH_3$ | H | 0 | $OCH_3$ |
| 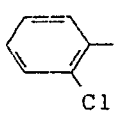 | 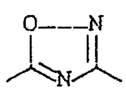 | O | 1 | H | H | $CH_3$ | H | 0 | $OCH_3$ |
| 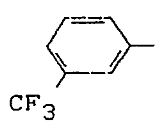 | 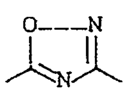 | O | 1 | H | H | $CH_3$ | H | 0 | $OCH_3$ |
| 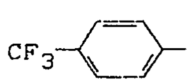 | 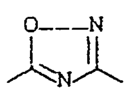 | O | 1 | H | H | $CH_3$ | H | 0 | $OCH_3$ |
| 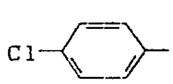 | 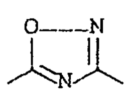 | O | 1 | H | H | $CH_3$ | H | 0 | $OCH_3$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 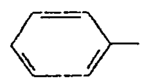 | 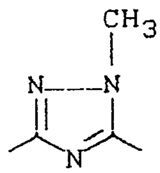 | O | 1 | H | H | $CH_3$ | H | O | $OCH_3$ |
| 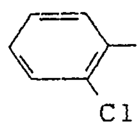 | 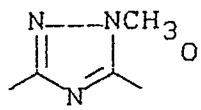 | O | 1 | H | H | $CH_3$ | H | O | $OCH_3$ |
| 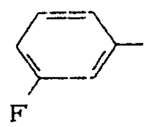 | 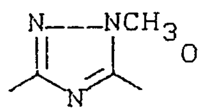 | O | 1 | H | H | $CH_3$ | H | O | $OCH_3$ |
| 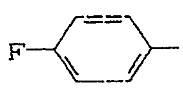 | 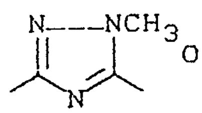 | O | 1 | H | H | $CH_3$ | H | O | $OCH_3$ |
| 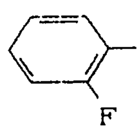 | 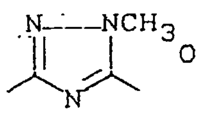 | O | 1 | H | H | $CH_3$ | H | O | $OCH_3$ |
| 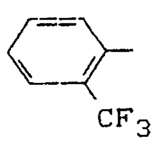 | 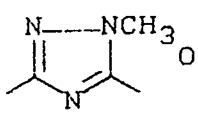 | O | 1 | H | H | $CH_3$ | H | O | $OCH_3$ |
| 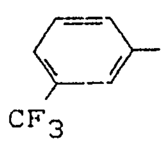 | 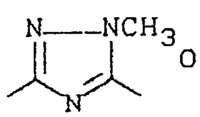 | O | 1 | H | H | $CH_3$ | H | O | $OCH_3$ |
| 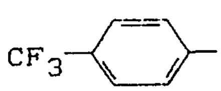 | 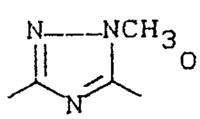 | O | 1 | H | H | $CH_3$ | H | O | $OCH_3$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 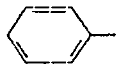 | 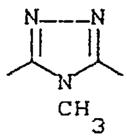 | O | 1 | H | H | $CH_3$ | H | 0 | $OCH_3$ |
| 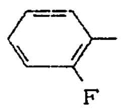 | 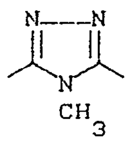 | O | 1 | H | H | $CH_3$ | H | 0 | $OCH_3$ |
| 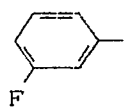 | 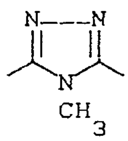 | O | 1 | H | H | $CH_3$ | H | 0 | $OCH_3$ |
| 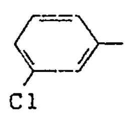 | 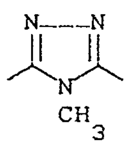 | O | 1 | H | H | $CH_3$ | H | 0 | $OCH_3$ |
| 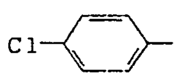 | 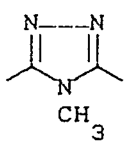 | O | 1 | H | H | $CH_3$ | H | 0 | $OCH_3$ |
| 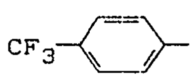 | 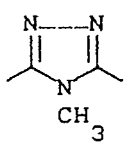 | O | 1 | H | H | $CH_3$ | H | 0 | $OCH_3$ |
| 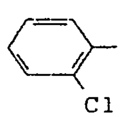 | 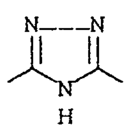 | O | 1 | H | H | $CH_3$ | H | 0 | $OCH_3$ |
| 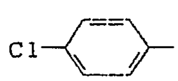 | 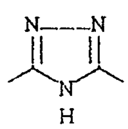 | O | 1 | H | H | $CH_3$ | H | 0 | $OCH_3$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 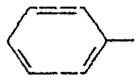 | 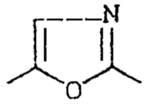 | O | 1 | H | H | $CH_3$ | H | 0 | $OCH_3$ |
| 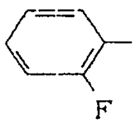 | 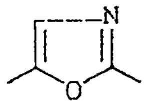 | O | 1 | H | H | $CH_3$ | H | 0 | $OCH_3$ |
| 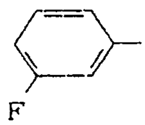 | 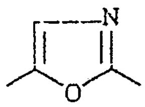 | O | 1 | H | H | $CH_3$ | H | 0 | $OCH_3$ |
| 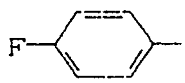 | 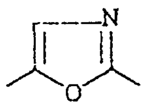 | O | 1 | H | H | $CH_3$ | H | 0 | $OCH_3$ |
| 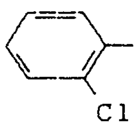 | 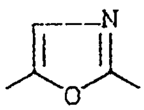 | O | 1 | H | H | $CH_3$ | H | 0 | $OCH_3$ |
| 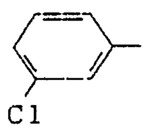 | 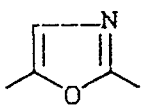 | O | 1 | H | H | $CH_3$ | H | 0 | $OCH_3$ |
| 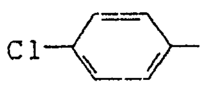 | 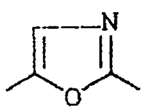 | O | 1 | H | H | $CH_3$ | H | 0 | $OCH_3$ |
| 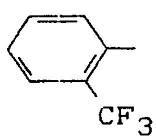 | 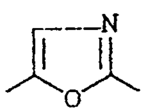 | O | 1 | H | H | $CH_3$ | H | 0 | OH |
| 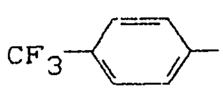 | 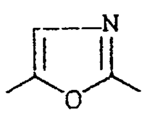 | O | 1 | H | H | $CH_3$ | H | 0 | OH |

Tabelle 1 - Fortsetzung

| Ar | Q | A | n | $X^1$ | $X^2$ | $R^1$ | $R^2$ | m | Y |
|---|---|---|---|---|---|---|---|---|---|
| 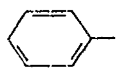 | 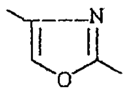 | O | 1 | H | H | $CH_3$ | H | 0 | $OCH_3$ |
| 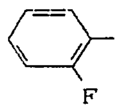 | 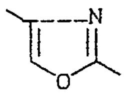 | O | 1 | H | H | $CH_3$ | H | 0 | $OCH_3$ |
| 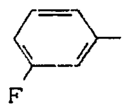 | 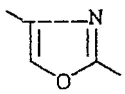 | O | 1 | H | H | $CH_3$ | H | 0 | $OCH_3$ |
| 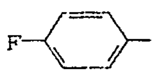 | 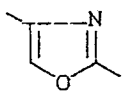 | O | 1 | H | H | $CH_3$ | H | 0 | $OCH_3$ |
| 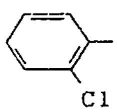 | 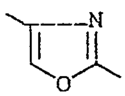 | O | 1 | H | H | $CH_3$ | H | 0 | $OCH_3$ |
| 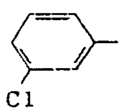 | 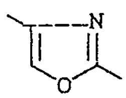 | O | 1 | H | H | $CH_3$ | H | 0 | $OCH_3$ |
| 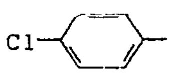 | 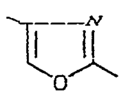 | O | 1 | H | H | $CH_3$ | H | 0 | $OCH_3$ |
| 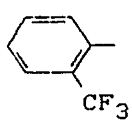 | 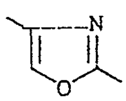 | O | 1 | H | H | $CH_3$ | H | 0 | OH |
| 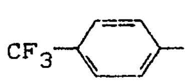 | 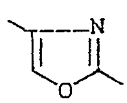 | O | 1 | H | H | $CH_3$ | H | 0 | OH |

Tabelle 1 - Fortsetzung

| Ar | Q | A | n | $X^1$ | $X^2$ | $R^1$ | $R^2$ | m | Y |
|---|---|---|---|---|---|---|---|---|---|
| 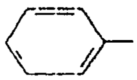 | 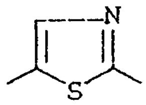 | O | 1 | H | H | $CH_3$ | H | 0 | $OCH_3$ |
| 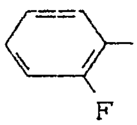 | 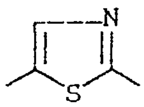 | O | 1 | H | H | $CH_3$ | H | 0 | $OCH_3$ |
| 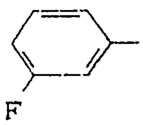 | 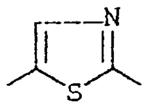 | O | 1 | H | H | $CH_3$ | H | 0 | $OCH_3$ |
| 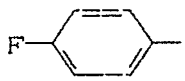 | 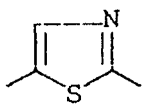 | O | 1 | H | H | $CH_3$ | H | 0 | $OC_2H_5$ |
| 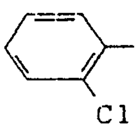 | 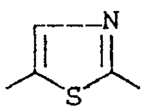 | O | 1 | H | H | $CH_3$ | H | 0 | $OC_2H_5$ |
| 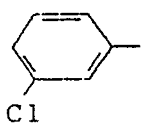 | 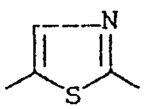 | O | 1 | H | H | $CH_3$ | H | 0 | $OC_2H_5$ |
| 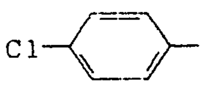 | 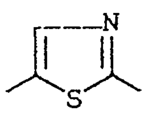 | O | 1 | H | H | $CH_3$ | H | 0 | $OC_2H_5$ |
| 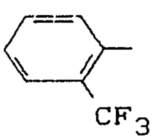 | 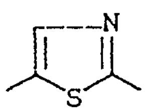 | O | 1 | H | H | $CH_3$ | H | 0 | $OC_2H_5$ |
| 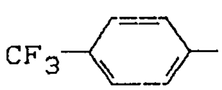 | 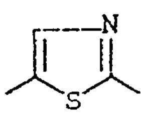 | O | 1 | H | H | $CH_3$ | H | 0 | $OC_2H_5$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 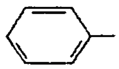 | 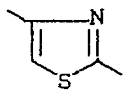 | O | 1 | H | H | $CH_3$ | H | 0 | $OCH_3$ |
| 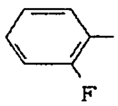 | 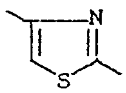 | O | 1 | H | H | $CH_3$ | H | 0 | $OCH_3$ |
| 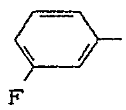 | 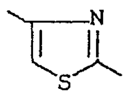 | O | 1 | H | H | $CH_3$ | H | 0 | $OCH_3$ |
| 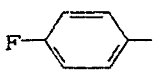 | 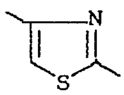 | O | 1 | H | H | $CH_3$ | H | 0 | $OC_2H_5$ |
| 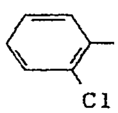 | 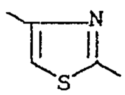 | O | 1 | H | H | $CH_3$ | H | 0 | $OC_2H_5$ |
| 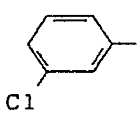 | 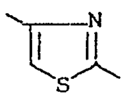 | O | 1 | H | H | $CH_3$ | H | 0 | $OC_2H_5$ |
| 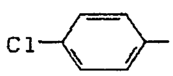 | 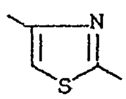 | O | 1 | H | H | $CH_3$ | H | 0 | $OC_2H_5$ |
| 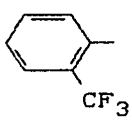 | 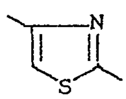 | O | 1 | H | H | $CH_3$ | H | 0 | $OC_2H_5$ |
| 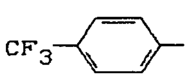 | 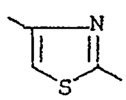 | O | 1 | H | H | $CH_3$ | H | 0 | $OC_2H_5$ |

<u>Tabelle 1</u> - Fortsetzung

| Ar | Q | A | n | $X^1$ | $X^2$ | $R^1$ | $R^2$ | m | Y |
|---|---|---|---|---|---|---|---|---|---|
| 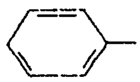 | 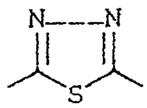 | - | 0 | H | H | $CH_3$ | H | 0 | $OCH_3$ |
| 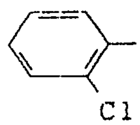 | 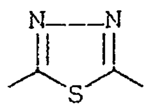 | - | 0 | H | H | $CH_3$ | H | 0 | $OCH_3$ |
| 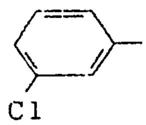 | 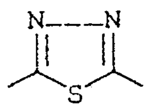 | - | 0 | H | H | $CH_3$ | H | 0 | $OC_2H_5$ |
| 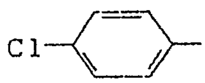 | 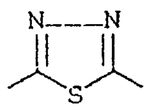 | - | 0 | H | H | $CH_3$ | H | 0 | $OC_2H_5$ |
| 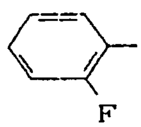 | 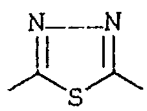 | - | 0 | H | H | $CH_3$ | H | 0 | $OC_2H_5$ |
| 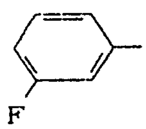 | 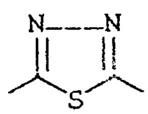 | - | 0 | H | H | $CH_3$ | H | 0 | $OC_2H_5$ |
| 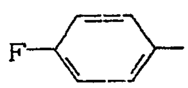 | 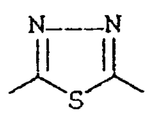 | - | 0 | H | H | $CH_3$ | H | 0 | OH |
| 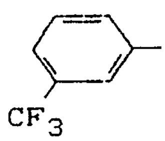 | 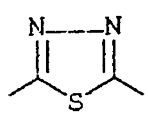 | - | 0 | H | H | $CH_3$ | H | 0 | OH |
| 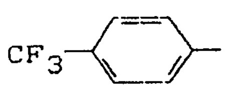 | 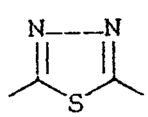 | - | 0 | H | H | $CH_3$ | H | 0 | OH |

<u>Tabelle 1</u> - Fortsetzung

| Ar | Q | A | n | $X^1$ | $X^2$ | $R^1$ | $R^2$ | m | Y |
|---|---|---|---|---|---|---|---|---|---|
| 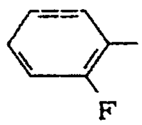 | 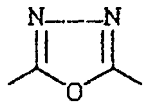 | - | 0 | H | H | $CH_3$ | H | 0 | $OCH_3$ |
| 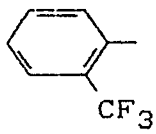 | 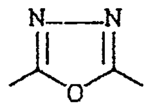 | - | 0 | H | H | $CH_3$ | H | 0 | $OCH_3$ |
| 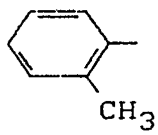 | 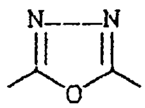 | - | 0 | H | H | $CH_3$ | H | 0 | $OC_2H_5$ |
| 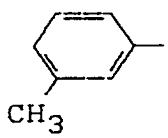 | 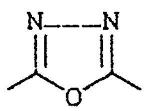 | - | 0 | H | H | $CH_3$ | H | 0 | $OC_2H_5$ |
| 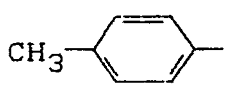 | 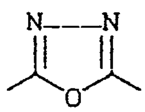 | - | 0 | H | H | $CH_3$ | H | 0 | $OC_2H_5$ |
| 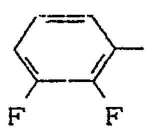 | 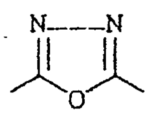 | - | 0 | H | H | $CH_3$ | H | 0 | $OC_2H_5$ |
| 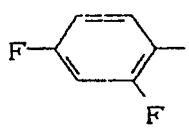 | 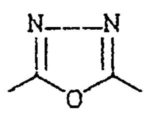 | - | 0 | H | H | $CH_3$ | H | 0 | OH |
| 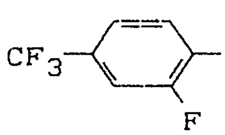 | 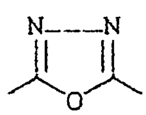 | - | 0 | H | H | $CH_3$ | H | 0 | OH |
| 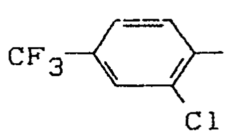 | 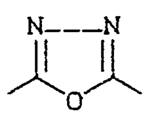 | - | 0 | H | H | $CH_3$ | H | 0 | OH |

Tabelle 1 - Fortsetzung

| Ar | Q | A | n | $X^1$ | $X^2$ | $R^1$ | $R^2$ | m | Y |
|---|---|---|---|---|---|---|---|---|---|
| 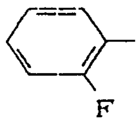 | 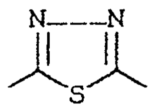 | O | 1 | H | H | H | H | 0 | $OC_2H_5$ |
| 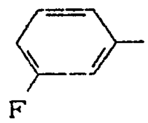 | 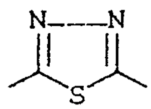 | O | 1 | H | H | H | H | 0 | $OC_2H_5$ |
| 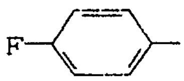 | 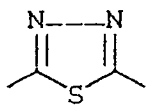 | O | 1 | H | H | H | H | 0 | $OCH_3$ |
| 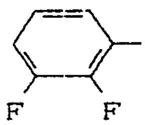 | 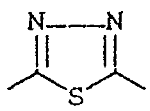 | O | 1 | H | H | H | H | 0 | $OCH_3$ |
| 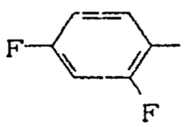 | 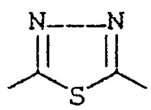 | O | 1 | H | H | H | H | 0 | $OCH_3$ |
| 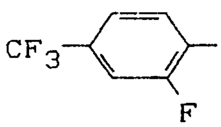 | 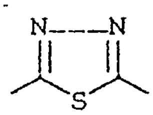 | O | 1 | H | H | H | H | 0 | $OCH_3$ |
| 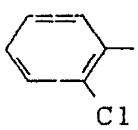 | 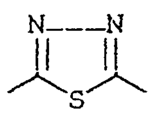 | O | 1 | H | H | H | H | 0 | $OCH_3$ |
| 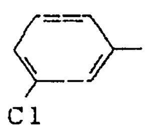 | 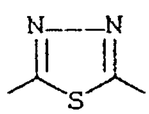 | O | 1 | H | H | H | H | 0 | $OCH_3$ |
| 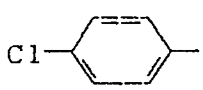 | 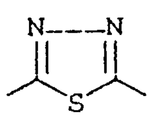 | O | 1 | H | H | H | H | 0 | $OCH_3$ |

<u>Tabelle 1</u> - Fortsetzung

| Ar | Q | A | n | $X^1$ | $X^2$ | $R^1$ | $R^2$ | m | Y |
|---|---|---|---|---|---|---|---|---|---|
| 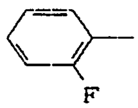 | 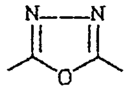 | O | 1 | H | H | H | H | 0 | $OC_2H_5$ |
| 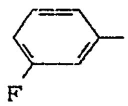 | 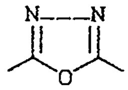 | O | 1 | H | H | H | H | 0 | $OC_2H_5$ |
| 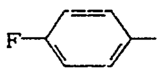 | 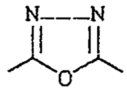 | O | 1 | H | H | H | H | 0 | $OCH_3$ |
| 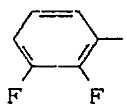 | 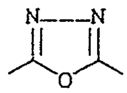 | O | 1 | H | H | H | H | 0 | $OCH_3$ |
| 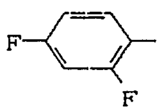 | 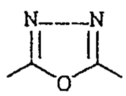 | O | 1 | H | H | H | H | 0 | $OCH_3$ |
| 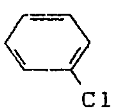 | 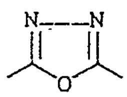 | O | 1 | H | H | H | H | 0 | $OCH_3$ |
| 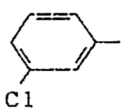 | 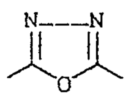 | O | 1 | H | H | H | H | 0 | $OCH_3$ |
| 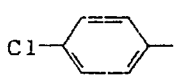 | 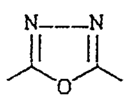 | O | 1 | H | H | H | H | 0 | $OCH_3$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 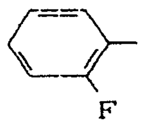 | 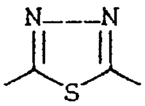 | O | 1 | H | H | $CH_3$ | $CH_3$ | 0 | OH |
| 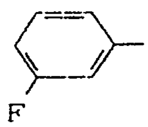 | 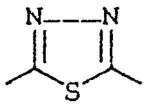 | O | 1 | H | H | $CH_3$ | H | 0 | OH |
| 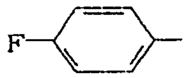 | 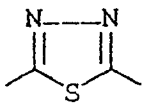 | O | 1 | H | H | $CH_3$ | H | 0 | OH |
| 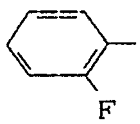 | 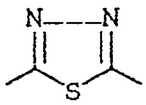 | O | 1 | H | H | $CH_3$ | H | 0 | $OC_2H_5$ |
| 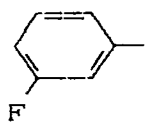 | 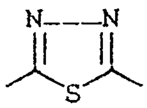 | O | 1 | H | H | $CH_3$ | H | 0 | $OC_2H_5$ |
| 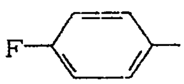 | 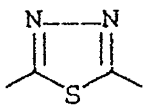 | O | 1 | H | H | $CH_3$ | H | 0 | $OC_2H_5$ |
| 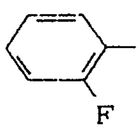 | 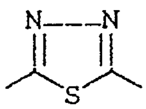 | O | 1 | H | H | $CH_3$ | H | 0 | Cl |
| 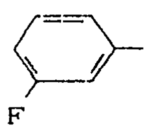 | 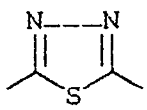 | O | 1 | H | H | $CH_3$ | H | 0 | Cl |
| 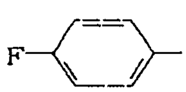 | 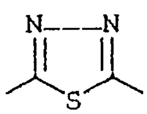 | O | 1 | H | H | $CH_3$ | H | 0 | Cl |

Tabelle 1 - Fortsetzung

| Ar | Q | A | n | $X^1$ | $X^2$ | $R^1$ | $R^2$ | m | Y |
|---|---|---|---|---|---|---|---|---|---|
| 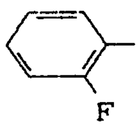 | 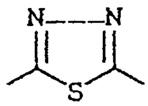 | O | 1 | H | H | $CH_3$ | H | 0 | $OnC_3H_7$ |
| 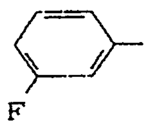 | 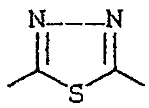 | O | 1 | H | H | $CH_3$ | H | 0 | $OnC_3H_7$ |
| 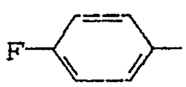 | 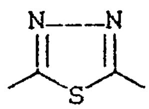 | O | 1 | H | H | $CH_3$ | H | 0 | $OnC_3H_7$ |
| 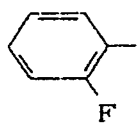 | 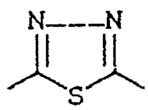 | O | 1 | H | H | $CH_3$ | H | 0 | $OiC_3H_7$ |
| 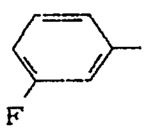 | 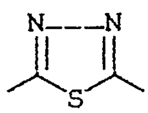 | O | 1 | H | H | $CH_3$ | H | 0 | $OiC_3H_7$ |
| 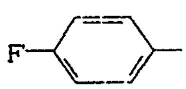 | 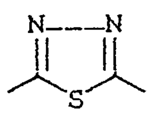 | O | 1 | H | H | $CH_3$ | H | 0 | $OiC_3H_7$ |
| 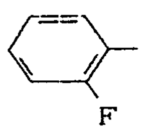 | 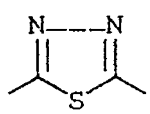 | O | 1 | H | H | $CH_3$ | H | 0 | $OnC_4H_9$ |
| 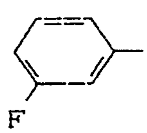 | 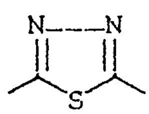 | O | 1 | H | H | $CH_3$ | H | 0 | $OnC_4H_9$ |
| 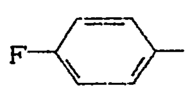 | 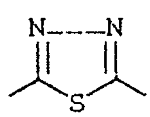 | O | 1 | H | H | $CH_3$ | H | 0 | $OnC_4H_9$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 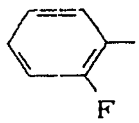 | 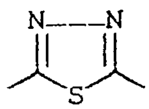 | O | 1 | H | H | $CH_3$ | H | 0 | $OsC_4H_9$ |
| 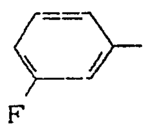 | 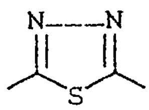 | O | 1 | H | H | $CH_3$ | H | 0 | $OsC_4H9$ |
| 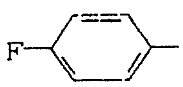 | 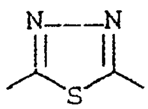 | O | 1 | H | H | $CH_3$ | H | 0 | $OsC_4H_9$ |
| 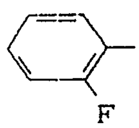 | 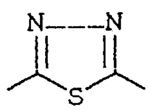 | O | 1 | H | H | $CH_3$ | H | 0 | $OCH_2-C{\equiv}CH$ |
| 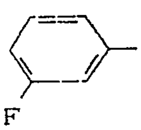 | 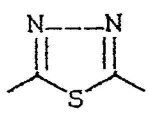 | O | 1 | H | H | $CH_3$ | H | 0 | $OCH_2-C{\equiv}CH$ |
| 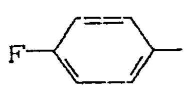 | 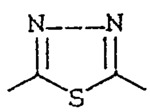 | O | 1 | H | H | $CH_3$ | H | 0 | $OCH_2-C{\equiv}CH$ |
| 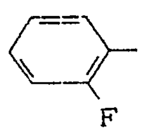 | 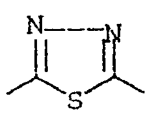 | O | 1 | H | H | $CH_3$ | H | 0 | $OCH_2CO_2C_2H_5$ |
| 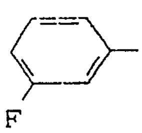 | 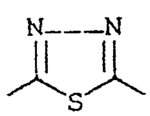 | O | 1 | H | H | $CH_3$ | H | 0 | $OCH_2CO_2C_2H_5$ |
| 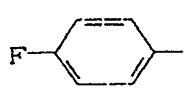 | 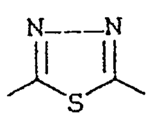 | O | 1 | H | H | $CH_3$ | H | 0 | $OCH_2CO_2C_2H_5$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 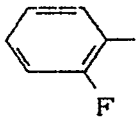 | 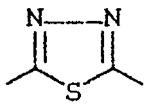 | O | 1 | H | H | $CH_3$ | H | 0 | $OCH_2C_6H_5$ |
| 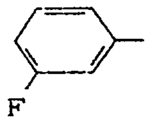 | 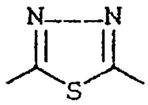 | O | 1 | H | H | $CH_3$ | H | 0 | $OCH_2C_6H_5$ |
| 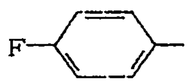 | 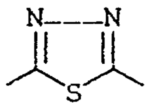 | O | 1 | H | H | $CH_3$ | H | 0 | $OCH_2C_6H_5$ |
| 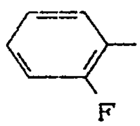 | 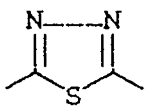 | O | 1 | H | H | $CH_3$ | H | 0 | $OCH_2OCH_2CH_2OCH_3$ |
| 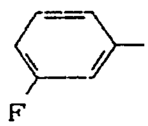 | 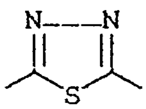 | O | 1 | H | H | $CH_3$ | H | 0 | $OCH_2OCH_2CH_2OCH_3$ |
| 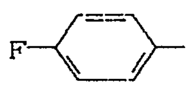 | 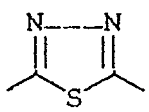 | O | 1 | H | H | $CH_3$ | H | 0 | $OCH_2OCH_2CH_2OCH_3$ |
| 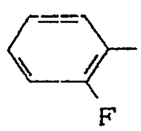 | 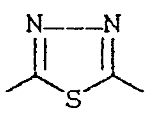 | O | 1 | H | H | $CH_3$ | H | 0 | $OCH_2CH_2OCH_2CH_3$ |
| 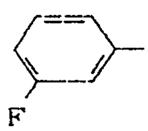 | 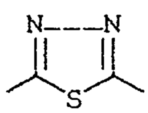 | O | 1 | H | H | $CH_3$ | H | 0 | $OCH_2CH_2OCH_2CH_3$ |
| 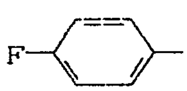 | 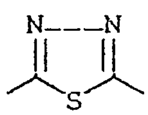 | O | 1 | H | H | $CH_3$ | H | 0 | $OCH_2CH_2OCH_2CH_3$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 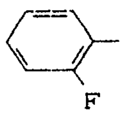 | 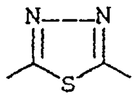 | O | 1 | H | H | $CH_3$ | H | 0 | $OCH_2CH_2OCH_2C_6H_4$-4Cl |
| 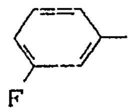 | 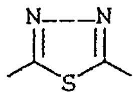 | O | 1 | H | H | $CH_3$ | H | 0 | $OCH_2CH_2OCH_2C_6H_4$-4Cl |
| 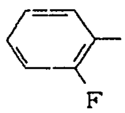 | 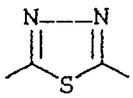 | O | 1 | H | H | $CH_3$ | H | 0 | $OCH_2CH_2OCH_2C_6H_4$-2F |
| 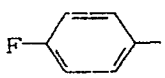 | 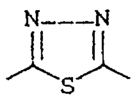 | O | 1 | H | H | $CH_3$ | H | 0 | $OCH_2CH_2OCH_2C_6H_4$-2F |
| 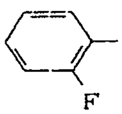 | 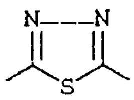 | O | 1 | H | H | $CH_3$ | H | 0 | $OCH_2CH_2SCH_2C_6H_5$ |
| 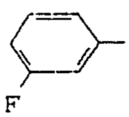 | 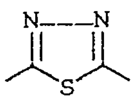 | O | 1 | H | H | $CH_3$ | H | 0 | $OCH_2CH_2SCH_2C_6H_5$ |
| 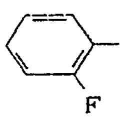 | 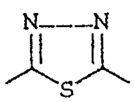 | O | 1 | H | H | $CH_3$ | H | 0 | $OCH(CH_3)OCH_2C_6H_5$ |
| 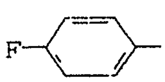 | 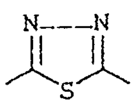 | O | 1 | H | H | $CH_3$ | H | 0 | $OCH(CH_3)OCH_2C_6H_5$ |
| 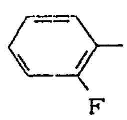 | 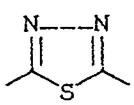 | O | 1 | H | H | $CH_3$ | H | 0 | $OCH_2CH_2CH_2OCH_2C_6H_5$ |
| 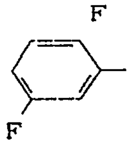 | 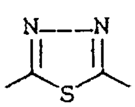 | O | 1 | H | H | $CH_3$ | H | 0 | $OCH_2CH_2CH_2OCH_2C_6H_5$ |

Tabelle 1 - Fortsetzung

| Ar | Q | A | n | $X^1$ | $X^2$ | $R^1$ | $R^2$ | m | Y |
|---|---|---|---|---|---|---|---|---|---|
| 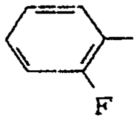 | 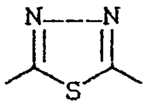 | 0 | 1 | H | H | $CH_3$ | H | 0 | $OCH_2$- 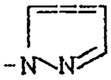 |
| 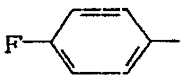 | 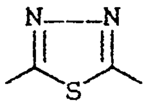 | 0 | 1 | H | H | $CH_3$ | H | 0 | $OCH_2$- 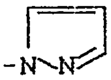 |
| 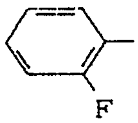 | 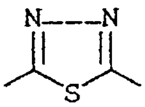 | 0 | 1 | H | H | $CH_3$ | H | 0 | $OCH_2CH_2$- 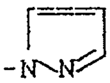 |
| 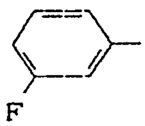 | 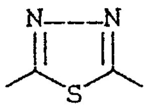 | 0 | 1 | H | H | $CH_3$ | H | 0 | $OCH_2CH_2$- 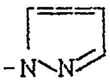 |
| 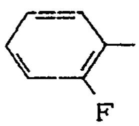 | 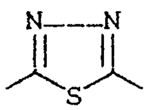 | 0 | 1 | H | H | $CH_3$ | H | 0 | $OCH_2$ 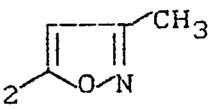 |
| 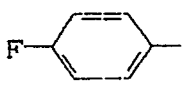 | 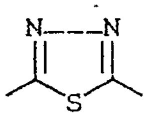 | 0 | 1 | H | H | $CH_3$ | H | 0 | $OCH_2$ 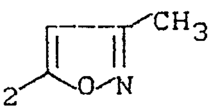 |
| 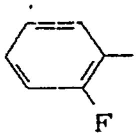 | 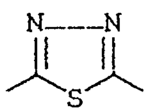 | 0 | 1 | H | H | $CH_3$ | H | 0 | 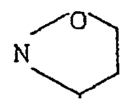 |
| 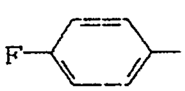 | 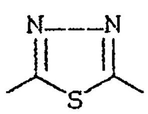 | 0 | 1 | H | H | $CH_3$ | H | 0 | 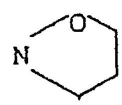 |

Tabelle 1 - Fortsetzung

| Ar | Q | A | n | $X^1$ | $X^2$ | $R^1$ | $R^2$ | m | Y |
|---|---|---|---|---|---|---|---|---|---|
| 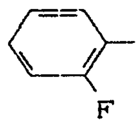 | 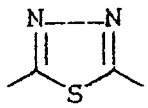 | O | 1 | H | H | $CH_3$ | H | O | $NH_2$ |
| 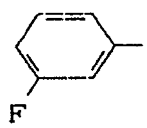 | 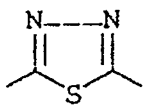 | O | 1 | H | H | $CH_3$ | H | O | $NH_2$ |
| 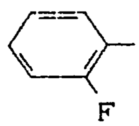 | 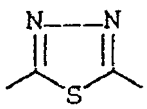 | O | 1 | H | H | $CH_3$ | H | O | $NHSO_2CH_3$ |
| 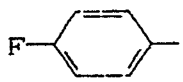 | 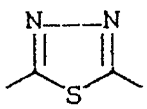 | O | 1 | H | H | $CH_3$ | H | O | $NHSO_2CH_3$ |
| 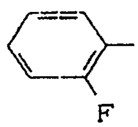 | 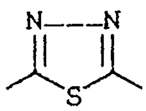 | O | 1 | H | H | $CH_3$ | H | O | $OCH_2Si(CH_3)_3$ |
| 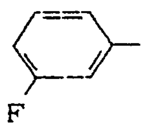 | 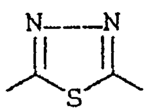 | O | 1 | H | H | $CH_3$ | H | O | $OCH_2Si(CH_3)_3$ |
| 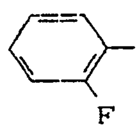 | 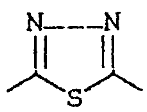 | O | 1 | H | H | $CH_3$ | H | O | $OCH_2$ 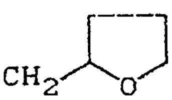 |
| 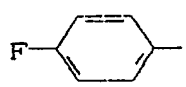 | 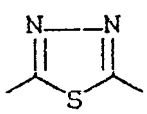 | O | 1 | H | H | $CH_3$ | H | O | $OCH_2$ 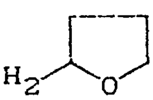 |

Tabelle 1 - Fortsetzung

| Ar | Q | A | n | $X^1$ | $X^2$ | $R^1$ | $R^2$ | m | Y |
|---|---|---|---|---|---|---|---|---|---|
| 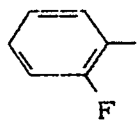 | 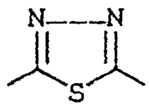 | O | 1 | H | H | $CH_3$ | H | 0 | $NHCH_2CO_2C_2H_5$ |
| 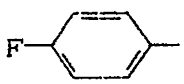 | 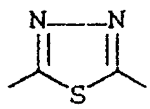 | O | 1 | H | H | $CH_3$ | H | 0 | $NHCH_2CO_2C_2H_5$ |
| 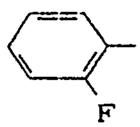 | 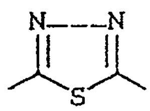 | O | 1 | H | H | $CH_3$ | H | 0 | ONa |
| 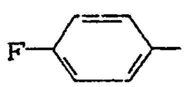 | 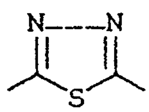 | O | 1 | H | H | $CH_3$ | H | 0 | ONa |
| 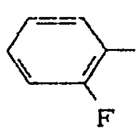 | 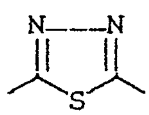 | O | 1 | H | H | $CH_3$ | H | 0 | $ONH_4$ |
| 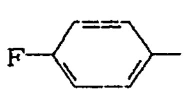 | 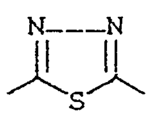 | O | 1 | H | H | $CH_3$ | H | 0 | $ONH_4$ |

Verwendet man für das erfindungsgemäße Verfahren (a) beispielsweise 2-Chlor-5-(4-fluor-phenyl)-1,3,4-oxadiazol und 2-(4-Hydroxy-phenoxy)-propionsäuremethylester als Ausgangsstoffe, so kann der Reaktionsablauf durch das folgende Formelschema wiedergegeben werden:

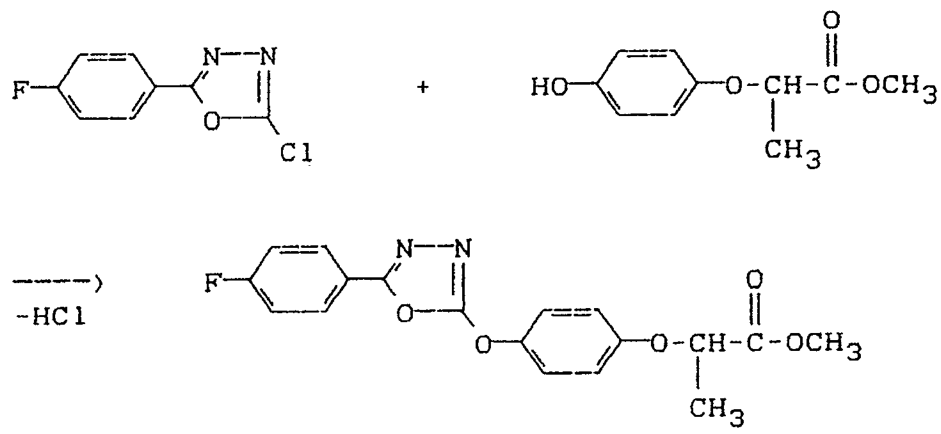


Verwendet man für das erfindungsgemäße Verfahren (b) beispielsweise 3-(4-Hydroxy-phenoxy)-5-(4-trifluormethyl-phenyl)-1,2,4-thiadiazol und 2-Methylsulfonyloxypropionsäureethylester als Ausgangsstoffe, so kann der Reaktionsablauf durch das folgende Formelschema wiedergegeben werden:

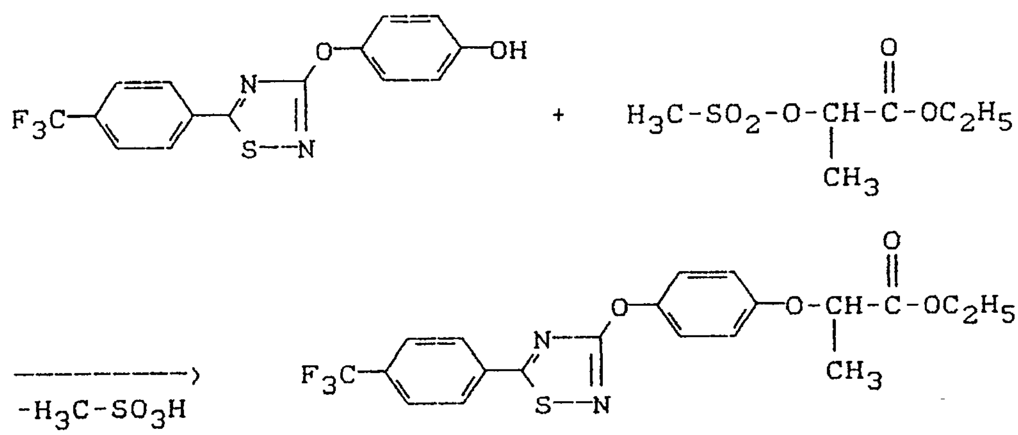


Die beim erfindungsgemäßen Verfahren (a) zur Herstellung von Verbindungen der Formel (I) als Ausgangsstoffe zu verwendenden Arylverbindungen sind durch die Formel (II) allgemein definiert.

In Formel (II) haben Ar und Q vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Ar und Q angegeben wurden.

$X^3$ steht vorzugsweise für Halogen, $C_1$-$C_4$-Alkylthio, $C_1$-$C_4$-Alkylsulfinyl, $C_1$-$C_4$-Alkylsulfonyl, $C_1$-$C_4$-Alkylsulfonyloxy, Phenylsulfonyloxy oder Tolylsulfonyloxy, insbesondere für Fluor, Chlor, Brom oder Methylsulfonyl.

Die Ausgangsstoffe der Formel (II) sind bekannt und/oder können nach an sich bekannten Verfahren hergestellt werden (vgl. J. Med. Chem. 31 (1988), 906-913; US-P 4454147; US-P 3959301; J. Prakt. Chem. 315 (1973), 185-188; FR-A 1575544; J Het. Chem. 10 (1973), 611-622 sowie die Herstellungsbeispiele).

Die beim erfindungsgemäßen Verfahren (a) zur Herstellung von Verbindungen der Formel (I) weiter als Ausgangsstoffe zu verwendenden Hydroxy- und Mercaptoaryloxycarbonylverbindungen sind durch die Formel (III) allgemein definiert.

In Formel (III) haben m, E, $X^1$, $X^2$, $R^1$, $R^2$ und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für m, E, $X^1$, $X^2$, $R^1$, $R^2$ und Y angegeben wurden.

$A^1$ steht für Sauerstoff oder Schwefel.

Die Ausgangsstoffe der Formel (III) sind bekannt und/oder können nach an sich bekannten Verfahren hergestellt werden (vgl. EP-A 82413; EP 180126).

Die beim erfindungsgemäßen Verfahren (b) zur Herstellung von Verbindungen der Formel (I) als

Ausgangsstoffe zu verwendenden Phenolderivate sind durch die Formel (IV) allgemein definiert.

In Formel (IV) haben n, Ar, Q, A, $X^1$ und $X^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, Ar, Q, A, $X^1$ und $X^2$ angegeben wurden.

Beispiele für die Ausgangsstoffe der Formel (IV) sind die Verbindungen der Tabelle 1, in denen der Rest

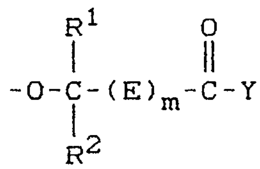

(gemäß Formel (I) dieser Verbindungen durch die Hydroxygruppe ersetzt ist.

Die Phenolderivate der Formel (IV) sind noch nicht aus der Literatur bekannt und sind Gegenstand der vorliegenden Patentanmeldung.

Für den Fall, daß in Formel (IV) n für die Zahl 1 und A für Sauerstoff oder Schwefel steht, erhält man die neuen Phenolderivate der Formel (IV), wenn man Arylverbindungen der Formel (II)

$Ar - Q - X^3$ (II)

in welcher

Ar, Q und $X^3$ die oben angegebenen Bedeutungen haben, mit Phenolen der Formel (VI)

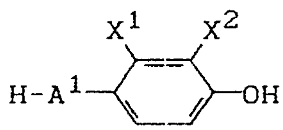

(VI)

in welcher

$X^1$ und $X^2$ die oben angegebenen Bedeutungen haben und $A^1$ für Sauerstoff oder Schwefel steht, in Gegenwart eines Säureakzeptors, wie z.B. Kaliumhydroxid und in Gegenwart eines Verdünnungsmittels, wie z.B. Dimethylsulfoxid, bei Temperaturen zwischen 0 °C und 150 °C umsetzt (vgl. die Herstellungsbeispiele).

Für den Fall, daß in Formel (IV) n für die Zahl 1 und A für Methylen oder n für die Zahl 0 steht, erhält man die neuen Phenolderivate der Formel (IV), wenn man Arylverbindungen der Formel (VII)

$Ar - Q^1$ (VII)

in welcher Ar die oben angegebene Bedeutung hat und $Q^1$ für einen organischen Rest mit ein oder zwei Kohlenstoffatomen, z.B. für CN, COOH, COCl, $CONH_2$, $CSNH_2$, $CONHNH_2$, $COOCH_3$, $C(NH)NH_2$, $C(NH)-NHNH_2$, $COCH_2Cl$ oder $COCH_2Br$, steht, mit Phenolen der Formel (VIII)

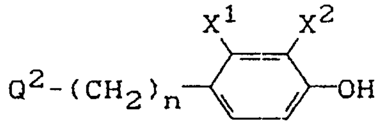

(VIII)

in welcher n, $X^1$ und $X^2$ die oben angegebenen Bedeutungen haben und $Q^2$ für einen organischen Rest mit ein oder zwei Kohlenstoffatomen, z.B. für CN, COOH, COCl, $CONH_2$, $CSNH_2$, $CONHNH_2$, $COOCH_3$, $C(NH)-NH_2$, $C(NH)NHNH_2$, $COCH_2Cl$ oder $COCH_2Br$, steht, umsetzt.

$Q^1$ in Formel (VII) und $Q^2$ in Formel (VIII) werden hierbei so gewählt, daß aus ihnen nach an sich

bekannten Verfahren der Heterocyclus Q konstruiert werden kann (vgl. die Herstellungsbeispiele).

Die beim erfindungsgemäßen Verfahren (b) zur Herstellung von Verbindungen der Formel (I) weiter als Ausgangsstoffe zu verwendenden Carbonsäurederivate sind durch die Formel (V) allgemein definiert.

In Formel (V) haben m, E, $R^1$, $R^2$ und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für m, E, $R^1$, $R^2$ und Y angegeben wurden;

$X^4$ steht vorzugsweise für Chlor, Brom, Iod, gegebenenfalls durch Fluor oder Chlor substituiertes $C_1$-$C_4$-Alkylsulfonyloxy oder gegebenenfalls durch Fluor, Chlor, Brom oder Methyl substituiertes Phenylsulfonyloxy, insbesondere für Chlor, Brom, Methylsulfonyloxy, Phenylsulfonyloxy oder 4-Methyl-phenylsulfonyloxy.

Als Beispiele für die Verbindungen der Formel (V) seien genannt:

α-Chlor-, α-Brom- und α-Iod-propionsäure-methylester, -ethylester, -propylester, -isopropylester, -butylester, -isobutylester und -sec-butylester, α-Methylsulfonyloxy-, α-Ethylsulfonyloxy-, α-Propylsulfonyloxy-, α-Butylsulfonyloxy-, α-Trifluormethylsulfonyloxy-, α-Phenylsulfonyloxy- und α-(4-Methyl-phenyl)-sulfonyloxy-propionsäure-methylester, -ethylester, -propylester, -butylester, -isopropylester, -isobutylester und -sec-butylester.

Unter den genannten Verbindungen der Formel (V) sind für den Fall, daß $R^1 \neq R^2$, jeweils die R-Isomeren, die S-Isomeren und die racemischen Gemische dieser Isomeren zu verstehen.

Die Ausgangsstoffe der Formel (V) sind bekannt und/oder können nach an sich bekannten Verfahren hergestellt werden (vgl. DE-OS 27 58 002, DE-OS 28 54 542).

Die erfindungsgemäßen Verfahren (a) und (b) zur Herstellung der neuen Aryloxycarbonylverbindungen der Formel (I) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei den erfindungsgemäßen Verfahren (a) und (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a) und (b) in einem größeren Bereich variiert werden Im allgemeinen arbeitet man bei Temperaturen zwischen 0 °C und 200 °C, vorzugsweise bei Temperaturen zwischen 20 °C und 150 °C.

Die erfindungsgemäßen Verfahren (a) und (b) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a) und (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) und (b) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in monokotylen und dikotylen Kulturen sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); 4-Amino-benzolsulfonyl-methylcarbonat (ASULAM); 2-Chlor-4-ethylamino-6-isopropylamino-1,3,5-triazin (ATRAZIN); 2-[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); 5-Amino-4-chlor-2-phenyl-2,3-dihydro-3-oxypyridazin (CHLORIDAZON); Ethyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 3,6-Dichlor-2-pyridincarbonsäure (CLOPYRALID); 2-Chlor-4-ethylamino-6-(3-cyanopropylamino)-1,3,5-triazin (CYANAZIN); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 3-Cyclohexyl-6-dimethylamino-1-methyl-1,3, 5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N, N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[-(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitrophenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenyl-carbamat (PROPHAM); 0-(6-Chlor-3-phenyl-pyridazin-4-yl)-S-octylthiocarbonat (PYRIDATE); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäuremethylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro ha.

Die Herstellung und die Verwendung der erfindungsgemäßen Wirkstoffe geht aus den nachfolgenden Beispielen hervor.

Herstellungsbeispiele:

Beispiel 1

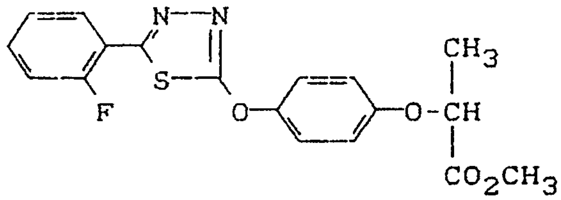

(Verfahren (a))

21,5 g (0,1 mol) 2-Chlor-5-(2-fluorphenyl)-1,3,4-thiadiazol werden in 1000 ml Acetonitril gelöst. Nach Zugabe von 30 g Kaliumcarbonat und 21,6 g (0,11 mol) (±)-2-(4-Hydroxyphenoxy)-propionsäuremethylester erhitzt man 36 h zum Rückfluß. Nach dem Erkalten wird das Acetonitril im Vakuum entfernt und der Rückstand zwischen Dichlormethan und Wasser verteilt. Die organische Phase wird abgetrennt, nacheinander mit verdünnter Natronlauge und Wasser gewaschen, getrocknet und eingeengt. Das so erhaltene Rohprodukt wird säulenchromatographisch an Kieselgel (Dichlormethan als Eluens) gereinigt.

Man erhält 22,6 g (60%) (±)-2-(4-[5-(2-Fluorphenyl)-1,3,4-thiadiazol-2-yloxy]-phenoxy)-propionsäuremethylester in Form eines gelben Feststoffs vom Schmelzpunkt 45° C.

Beispiel 2

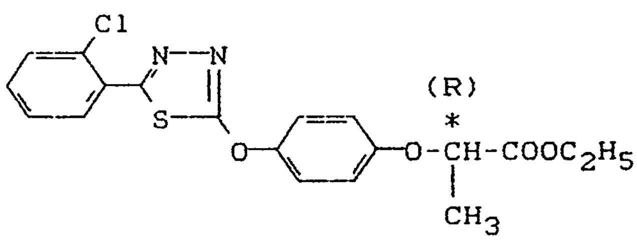

(Verfahren (b))

Eine Mischung aus 15,2 g (0,05 mol) 2-(4-Hydroxy-phenoxy)-5-(2-chlor-phenyl)-1,3,4-thiadiazol, 6,9 g Kaliumcarbonat und 95 ml Acetonitril wird auf 60° C erwärmt und unter Rühren wird eine Lösung von 13,6 g (0,05 mol) (S)-α-(4-Methyl-phenyl)-sulfonyloxy-propionsäure-ethylester in 20 ml Acetonitril tropfenweise dazugegeben.

Das Reaktionsgemisch wird dann 5 Stunden unter Rückfluß erhitzt und anschließend eingeengt. Aus dem Rückstand wird das Produkt mit Chloroform extrahiert, die organische Phase nacheinander mit 2N-Natronlauge, Wasser, 2N-Salzsäure und wieder Wasser geschüttelt, mit Natriumsulfat getrocknet und filtriert. Vom Filtrat wird das Lösungsmittel im Wasserstrahlvakuum sorgfältig abdestilliert.

Man erhält 14,5 g (72% der Theorie) (R)-2-(4-[5-(2-Chlor-phenyl)-1,3,4-thiadiazol-2-yl-oxy]-phenoxy)-propionsäure-ethylester vom Brechungsindex $n_D^{20}$ = 1,5974.

Analog zu den Beispielen 1 und 2 sowie entsprechend der allgemeinen Beschreibung der erfindungsgemäßen Herstellungsverfahren können beispielsweise auch die in der nachstehenden Tabelle 5 aufgeführten Verbindungen der Formel (I) hergestellt werden.

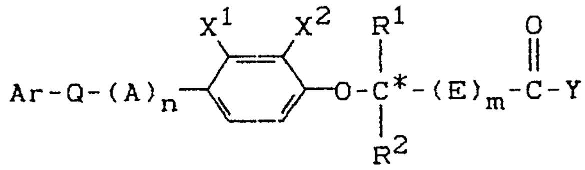 (I)*

<u>Tabelle 2:</u> Herstellungsbeispiele für die Verbindungen der Formel (I)

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 3 | 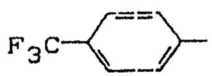 | 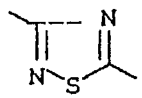 | O | 1 | H | H | $CH_3$ | H | 0 | $OCH_3$ | mp.:70° C |
| 4 | 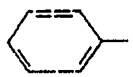 | 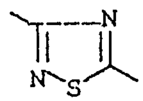 | O | 1 | H | H | $CH_3$ | H | 0 | $OC_2H_5$ | ($n_D^{20}$ : 1,5838) *) |
| 5 | 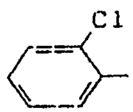 | 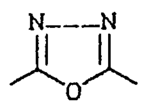 | - | 0 | H | H | $CH_3$ | H | 0 | $OC_2H_5$ | mp.:83° C |
| 6 | 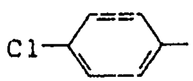 | 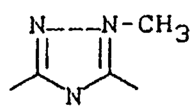 | O | 1 | H | H | $CH_3$ | H | 0 | $OC_2H_5$ | mp.:105° C |
| 7 | 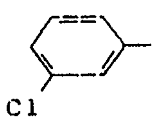 | 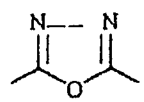 | - | 0 | H | H | $CH_3$ | H | 0 | $OC_2H_5$ | mp.:64° C |
| 8 | 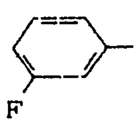 | 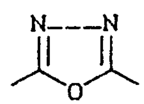 | - | 0 | H | H | $CH_3$ | H | 0 | $OC_2H_5$ | mp.:76° C |

*) an dem mit * markierten C-Atom R-konfiguriert (vgl. Beispiel 2)

<u>Tabelle 2</u> - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 9 | 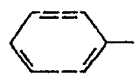 | 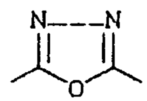 | - | 0 | H | H | $CH_3$ | H | 0 | $OC_2H_5$ | mp.: 80° C |
| 10 | 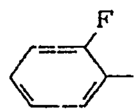 | 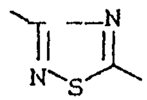 | O | 1 | H | H | $CH_3$ | H | 0 | $OCH_3$ | δ = 1,65<br>3,79<br>4,79 |
| 11 | 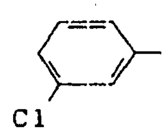 | 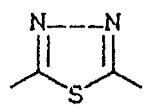 | O | 1 | H | H | $CH_3$ | H | 0 | $OCH_3$ | mp.: 63° C |
| 12 | 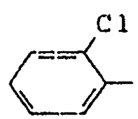 | 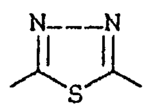 | O | 1 | H | H | $CH_3$ | H | 0 | $OCH_3$ | mp.: 65° C |
| 13 | 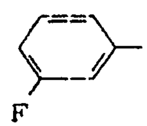 | 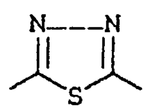 | O | 1 | H | H | $CH_3$ | H | 0 | $OCH_3$ | mp.: 42° C |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 14 | 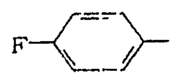 | 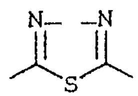 | O | 1 | H | H | $CH_3$ | H | 0 | $OCH_3$ | mp.:31° C |
| 15 | 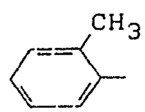 | 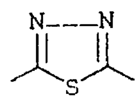 | O | 1 | H | H | $CH_3$ | H | 0 | $OCH_3$ | δ = 1,64, 2,57, 3,78, 4,76 |
| 16 | 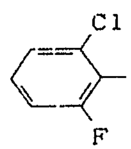 | 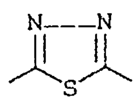 | O | 1 | H | H | $CH_3$ | H | 0 | $OCH_3$ | δ = 6,93, 7,14, 7,32, 7,41 |
| 17 | 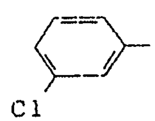 | 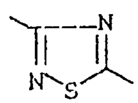 | O | 1 | H | H | $CH_3$ | H | 0 | $OCH_3$ | δ = 1,66, 3,80, 4,79 |
| 18 | 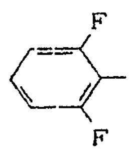 | 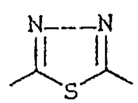 | O | 1 | H | H | $CH_3$ | H | 0 | $OCH_3$ | δ = 6,93, 7,06, 7,30, 7,44 |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 19 | 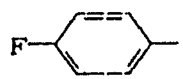 | 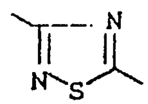 | O | 1 | H | H | $CH_3$ | H | 0 | $OCH_3$ | δ = 1,66, 3,79, 4,79 |
| 20 | 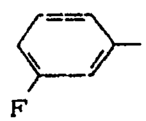 | 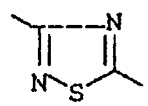 | O | 1 | H | H | $CH_3$ | H | 0 | $OCH_3$ | δ = 1,66, 3,80, 4,79 |
| 21 | 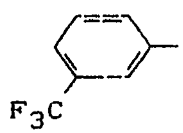 | 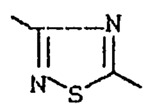 | O | 1 | H | H | $CH_3$ | H | 0 | $OCH_3$ | δ = 1,66, 3,80, 4,79 |
| 22 | 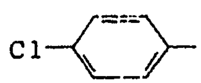 | 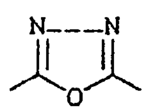 | - | 0 | H | H | $CH_3$ | H | 0 | $OC_2H_5$ | mp.:69° C |
| 23 | 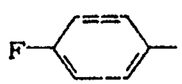 | 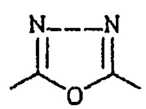 | - | 0 | H | H | $CH_3$ | H | 0 | $OC_2H_5$ | δ = 7,02, 7,23, 8,05, 8,12 |
| 24 | 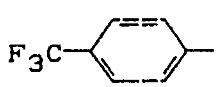 | 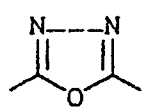 | - | 0 | H | H | $CH_3$ | H | 0 | $OC_2H_5$ | δ = 7.02, 7,80, 8,08, 8,25 |

<u>Tabelle 2</u> - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 25 | 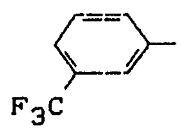 | 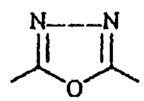 | - | 0 | H | H | $CH_3$ | H | 0 | $OC_2H_5$ | δ = 7,03, 7,70, 7,82, 8,11, 8,3-8,4 |
| 26 | 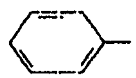 | 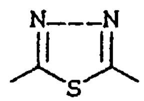 | O | 1 | H | H | $CH_3$ | H | 0 | $OC_2H_5$ | ($n_D^{20}$ : 1,5920) *) |
| 27 | 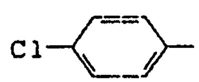 | 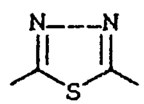 | O | 1 | H | H | $CH_3$ | H | 0 | $OC_2H_5$ | mp.:55° C*) |
| 28 | 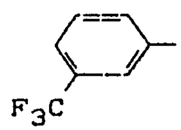 | 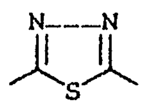 | O | 1 | H | H | $CH_3$ | H | 0 | $OC_2H_5$ | ($n_D^{20}$ : 1,5567) *) |
| 29 | 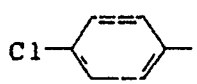 | 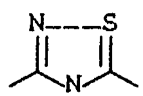 | O | 1 | H | H | $CH_3$ | H | 0 | $OCH_3$ | (δ=1,66,3,80, 4,79) |
| 30 | 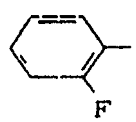 | 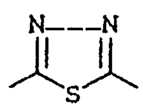 | O | 1 | H | H | $CH_3$ | H | 0 | $OC_2H_5$ | mp.:32° C *) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 31 | 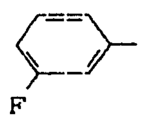 | 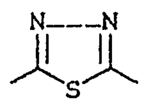 | O | 1 | H | H | $CH_3$ | H | 0 | $OC_2H_5$ | δ=6,93,7,16, 7,28,7,41, 7,58 *) |
| 32 | 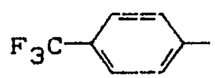 | 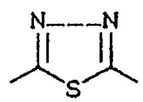 | O | 1 | H | H | $CH_3$ | H | 0 | $OCH_3$ | mp.:55° C |
| 33 | 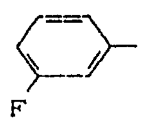 | 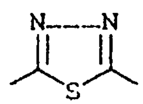 | O | 1 | H | H | H | H | 0 | $OC_2H_5$ | mp.:32° C *) |
| 34 | 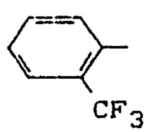 | 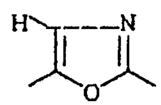 | O | 1 | H | H | $CH_3$ | H | 0 | $OC_2H_5$ | (amorph) |
| 35 | 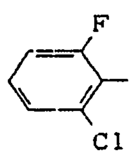 | 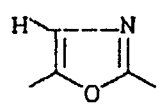 | O | 1 | H | H | $CH_3$ | H | 0 | $OC_2H_5$ | (amorph) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 36 | 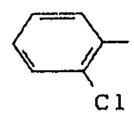 | 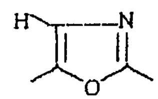 | O | 1 | H | H | $CH_3$ | H | 0 | $OC_2H_5$ | mp.: 49° C |
| 37 | 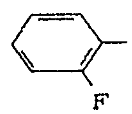 | 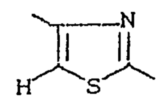 | O | 1 | H | H | $CH_3$ | H | 0 | $OC_2H_5$ | (amorph) |
| 38 | 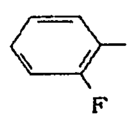 | 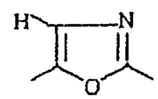 | O | 1 | H | H | $CH_3$ | H | 0 | $OC_2H_5$ | (amorph) |
| 39 | 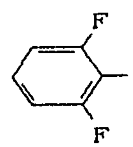 | 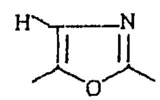 | O | 1 | H | H | $CH_3$ | H | 0 | $OC_2H_5$ | (amorph) |
| 40 | 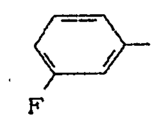 | 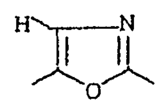 | O | 1 | H | H | $CH_3$ | H | 0 | $OC_2H_5$ | mp.: 50° C |

<u>Tabelle 2</u> - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 41 | 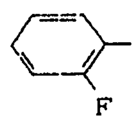 | 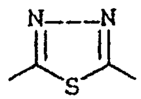 | O | 1 | H | H | H | H | 0 | $OC_2H_5$ | mp.: 52° C |
| 42 | 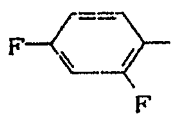 | 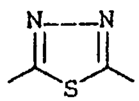 | O | 1 | H | H | $CH_3$ | H | 0 | $OC_2H_5$ | (Öl)*) |
| 43 | 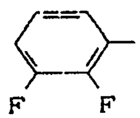 | 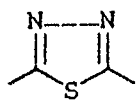 | O | 1 | H | H | $CH_3$ | H | 0 | $OC_2H_5$ | (Öl)*) |
| 44 | 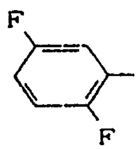 | 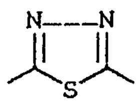 | O | 1 | H | H | $CH_3$ | H | 0 | $OC_2H_5$ | δ = 8,01; 7,28; 7,15; 6,94 *) |
| 45 | 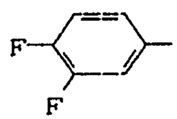 | 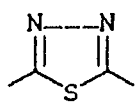 | O | 1 | H | H | $CH_3$ | H | 0 | $OC_2H_5$ | δ = 7,73; 7,52; 7,27; 7,22; 6,93 *) |

<u>Tabelle 2</u> - Fortsetzung

| Bsp.-Nr. | Ar | Q | A | n | $X^1$ | $X^2$ | $R^1$ | $R^2$ | m | Y | physikalische Daten | |
|---|---|---|---|---|---|---|---|---|---|---|---|---|
| 46 | 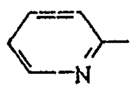 | 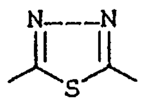 | O | 1 | H | H | $CH_3$ | H | 0 | $OC_2H_5$ | δ = 8,57; 8,22; 7,83; 7,36; 7,28; 6,92 | *) |
| 47 | 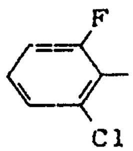 | 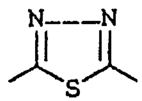 | O | 1 | H | H | $CH_3$ | H | 0 | $OC_2H_5$ | (Öl) | *) |
| 48 | 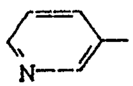 | 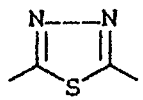 | O | 1 | H | H | $CH_3$ | H | 0 | $OC_2H_5$ | mp.: 58° C | *) |
| 49 | 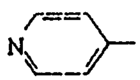 | 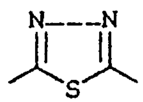 | O | 1 | H | H | $CH_3$ | H | 0 | $OC_2H_5$ | δ = 8,76; 7,80; 7,29; 6,94 | *) |
| 50 | 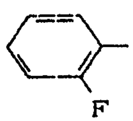 | 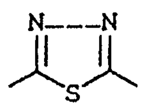 | O | 1 | H | H | $CH_3$ | H | 0 | OH | mp.: 105° C | *) |

Tabelle 2 - Fortsetzung

| Bsp.-Nr. | Ar | Q | A | n | $X^1$ | $X^2$ | $R^1$ | $R^2$ | m | Y | physikalische Daten | |
|---|---|---|---|---|---|---|---|---|---|---|---|---|
| 51 | 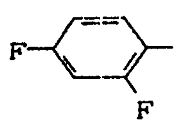 | 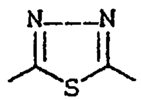 | O | 1 | H | H | $CH_3$ | H | 0 | OH | mp.: 110° C | |
| 52 | 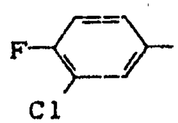 | 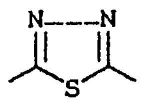 | O | 1 | H | H | $CH_3$ | H | 0 | $OC_2H_5$ | δ = 8,01; 7,80; 7,45; 7,39; 7,12 | *) |
| 53 | 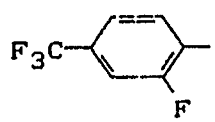 | 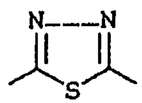 | O | 1 | H | H | $CH_3$ | H | 0 | $OC_2H_5$ | mp.: 44° C | *) |
| 54 | 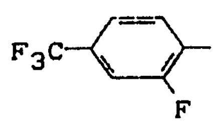 | 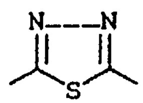 | O | 1 | H | H | $CH_3$ | H | 0 | OH | mp.: 84° C | *) |
| 55 | 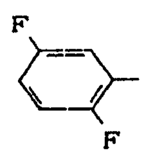 | 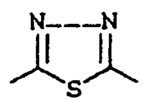 | O | 1 | H | H | $CH_3$ | H | 0 | OH | mp.: 84° C | *) |

Tabelle 2 - Fortsetzung

| Bsp.-Nr. | Ar | Q | A | n | $X^1$ | $X^2$ | $R^1$ | $R^2$ | m | Y | physikalische Daten | |
|---|---|---|---|---|---|---|---|---|---|---|---|---|
| 56 | 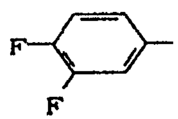 | 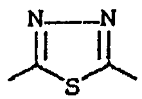 | O | 1 | H | H | $CH_3$ | H | 0 | OH | mp.: 118° C | *) |
| 57 | 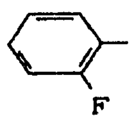 | 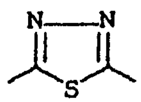 | O | 1 | H | H | $CH_3$ | H | 0 | $-OCH_2-$ 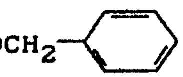 | (Öl) | |
| 58 | 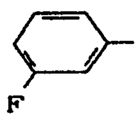 | 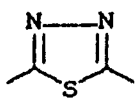 | O | 1 | H | H | $CH_3$ | H | 0 | OH | mp.: 98° C | *) |
| 59 | 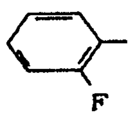 | 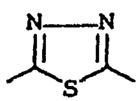 | O | 1 | H | H | $CH_3$ | H | 0 | $OCH_3$ | $\delta$ = 4,78; 3,78; 1,64 | *) |
| 60 | 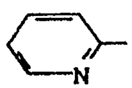 | 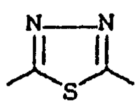 | O | 1 | H | H | $CH_3$ | H | 0 | OH | mp.: 198° C | *) |

Tabelle 2 - Fortsetzung

| Bsp.-Nr. | Ar | Q | A | n | $X^1$ | $X^2$ | $R^1$ | $R^2$ | m | Y | physikalische Daten | |
|---|---|---|---|---|---|---|---|---|---|---|---|---|
| 61 | 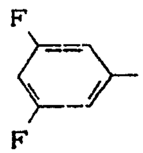 | 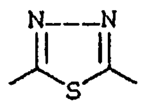 | O | 1 | H | H | $CH_3$ | H | O | OH | mp.: 100° C | *) |
| 62 | 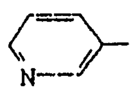 | 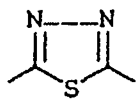 | O | 1 | H | H | $CH_3$ | H | O | OH | mp.: 172° C | *) |
| 63 | 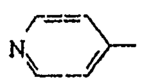 | 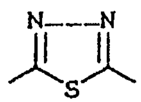 | O | 1 | H | H | $CH_3$ | H | O | OH | mp.: 187° C | *) |
| 64 | 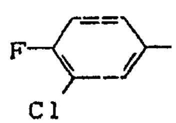 | 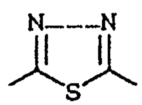 | O | 1 | H | H | $CH_3$ | H | O | OH | (amorph) | *) |
| 65 | 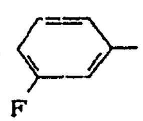 | 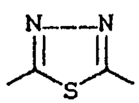 | O | 1 | H | H | $CH_3$ | H | O | $-OCH_2-$ 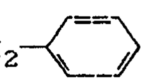 | mp.: 75° C | *) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 66 | 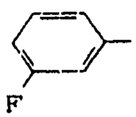 | 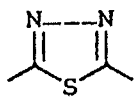 | O | 1 | H | H | $CH_3$ | H | 0 | $OCH_3$ | $n_D^{20}$ = 1,5907 *) |
| 67 | 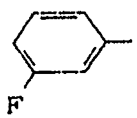 | 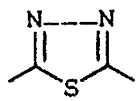 | O | 1 | H | H | $CH_3$ | H | 0 | $-OCH_2COOC_2H_5$ | $n_D^{20}$ = 1,5664*) |
| 68 | 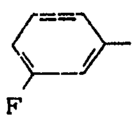 | 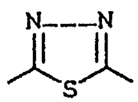 | O | 1 | H | H | $CH_3$ | H | 0 | $-OCH_2OCH_2CH_2OCH_3$ | $n_D^{20}$ = 1,5552*) |
| 69 | 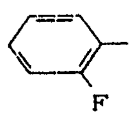 | 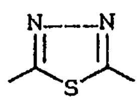 | O | 1 | H | H | $CH_3$ | H | 0 | $-OCH_2COOC_2H_5$ | δ = 4,87; 4,70; 4,23; 1,72; 1,27 *) |
| 70 | 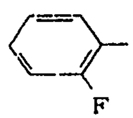 | 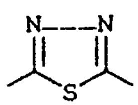 | O | 1 | H | H | $CH_3$ | H | 0 | $-OCH_2OCH_2CH_2OCH_3$ | δ = 8,30; 7,46; 7,28; 7,19; 6,94 |

Tabelle 2 - Fortsetzung

| Bsp.-Nr. | Ar | Q | A | n | $X^1$ | $X^2$ | $R^1$ | $R^2$ | m | Y | physikalische Daten | |
|---|---|---|---|---|---|---|---|---|---|---|---|---|
| 71 | 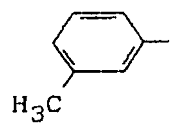 | 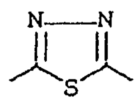 | O | 1 | H | H | $CH_3$ | H | 0 | $OC_2H_5$ | (Öl) | *) |
| 72 | 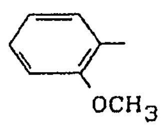 | 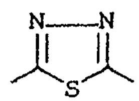 | O | 1 | H | H | $CH_3$ | H | 0 | $OC_2H_5$ | mp.: 90° C | *) |
| 73 | 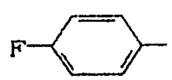 | 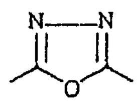 | O | 1 | H | H | $CH_3$ | H | 0 | $OC_2H_5$ | mp.: 61° C | *) |
| 74 | 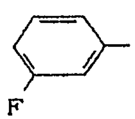 | 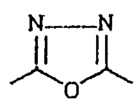 | O | 1 | H | H | $CH_3$ | H | 0 | $OC_2H_5$ | (Öl) | *) |
| 75 | 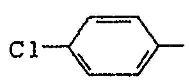 | 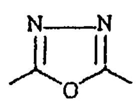 | O | 1 | H | H | $CH_3$ | H | 0 | $OC_2H_5$ | mp.: 31° C | *) |

Tabelle 2 - Fortsetzung

| Bsp.-Nr. | Ar | Q | A | n | $X^1$ | $X^2$ | $R^1$ | $R^2$ | m | Y | physikalische Daten | |
|---|---|---|---|---|---|---|---|---|---|---|---|---|
| 76 | 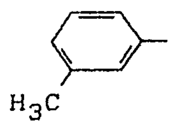 | 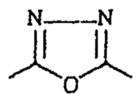 | O | 1 | H | H | $CH_3$ | H | 0 | $OC_2H_5$ | (Öl) | *) |
| 77 | 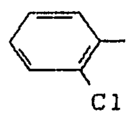 | 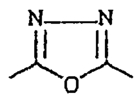 | O | 1 | H | H | $CH_3$ | H | 0 | $OC_2H_5$ | (Öl) | *) |
| 78 | 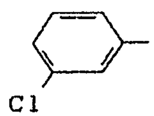 | 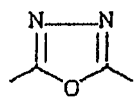 | O | 1 | H | H | $CH_3$ | H | 0 | $OC_2H_5$ | mp.: 28° C | *) |
| 79 | 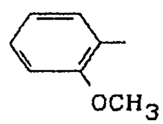 | 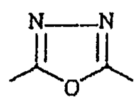 | O | 1 | H | H | $CH_3$ | H | 0 | $OC_2H_5$ | (Öl) | *) |
| 80 | 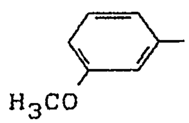 | 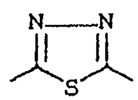 | O | 1 | H | H | $CH_3$ | H | 0 | $OC_2H_5$ | (amorph) | *) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 81 | 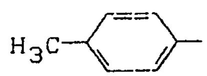 | 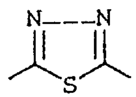 | O | 1 | H | H | $CH_3$ | H | 0 | $OC_2H_5$ | mp.: 46° C *) |
| 82 | 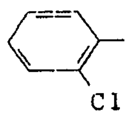 | 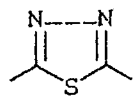 | O | 1 | H | H | $CH_3$ | H | 0 | OH | mp.: 92° C *) |
| 83 | 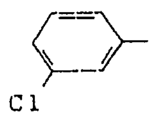 | 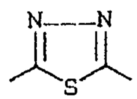 | O | 1 | H | H | $CH_3$ | H | 0 | OH | mp.: 66° C *) |
| 84 | 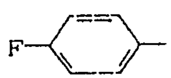 | 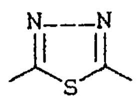 | O | 1 | H | H | $CH_3$ | H | 0 | OH | mp.: 103° C *) |
| 85 | 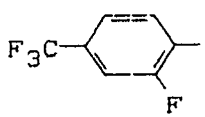 | 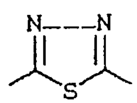 | O | 1 | H | H | $CH_3$ | H | 0 | $OCH_3$ | (Öl) *) |
| 86 | 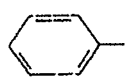 | 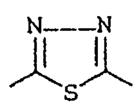 | O | 1 | H | H | $CH_3$ | H | 0 | OH | mp.: 105° C *) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 87 | 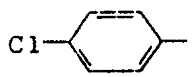 | 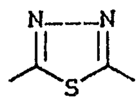 | O | 1 | H | H | $CH_3$ | H | 0 | OH | mp.: 98° C *) |
| 88 | 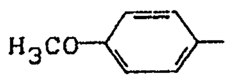 | 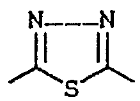 | O | 1 | H | H | $CH_3$ | H | 0 | OH | mp.: 79° C *) |
| 89 | 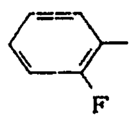 | 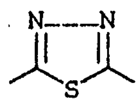 | O | 1 | H | H | $CH_3$ | H | 0 | $OCH_2CH_2CH_3$ | δ = 4,77; 4,14; 1,67; 0,90 |
| 90 | 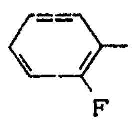 | 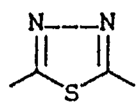 | O | 1 | H | H | $CH_3$ | H | 0 | $-OCH_2C{\equiv}CH$ | δ = 4,80; 4,78; 2,50; 1,68 |
| 91 | 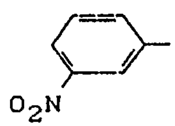 | 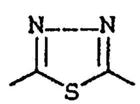 | O | 1 | H | H | $CH_3$ | H | 0 | OH | mp.: 53° C *) |
| 92 | 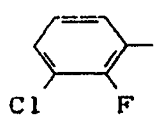 | 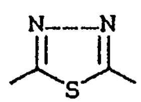 | O | 1 | H | H | $CH_3$ | H | 0 | OH | mp.: 105° C *) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 93 | 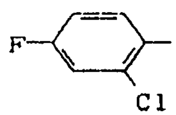 | 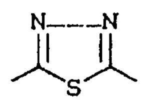 | O | 1 | H | H | $CH_3$ | H | 0 | OH | mp.: 127° C *) |
| 94 | 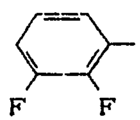 | 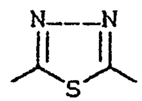 | O | 1 | H | H | $CH_3$ | H | 0 | OH | mp.: 135° C *) |
| 95 | 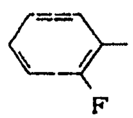 | 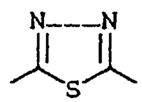 | O | 1 | H | H | $CH_3$ | H | 0 | $OCH(CH_3)_2$ | δ = 5,10; 4,72; 1,63; 1,29; 1,20 |
| 96 | 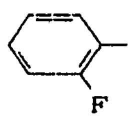 | 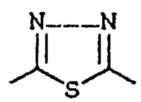 | O | 1 | H | H | $CH_3$ | H | 0 | $OCH_2CH_2CH_2CH_3$ | (Öl) |
| 97 | 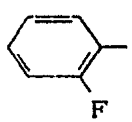 | 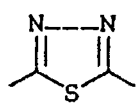 | O | 1 | H | H | $CH_3$ | H | 0 | $OCHCH_2CH_3$<br>$CH_3$ | (Öl) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 98 | 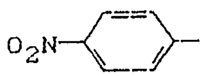 | 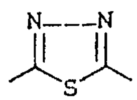 | O | 1 | H | H | $CH_3$ | H | 0 | $OC_2H_5$ | mp.: 92° C *) |
| 99 | 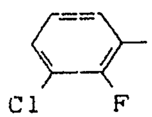 | 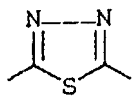 | O | 1 | H | H | $CH_3$ | H | 0 | $OC_2H_5$ | (Öl) *) |
| 100 | 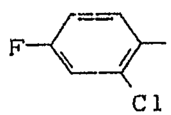 | 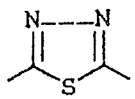 | O | 1 | H | H | $CH_3$ | H | 0 | $OC_2H_5$ | (Öl) *) |
| 101 | 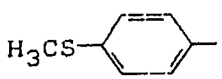 | 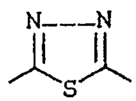 | O | 1 | H | H | $CH_3$ | H | 0 | $OC_2H_5$ | (Öl) *) |
| 102 | 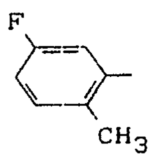 | 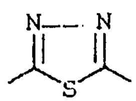 | O | 1 | H | H | $CH_3$ | H | 0 | $OC_2H_5$ | δ = 4,73; 4,24; 2,51; 1,63; 1,28 *) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 103 | 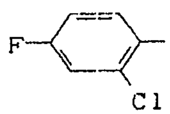 | 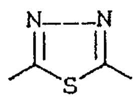 | O | 1 | H | H | $CH_3$ | H | 0 | $OCH_3$ | mp.: 55° C *) |
| 104 | 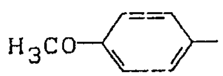 | 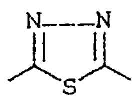 | O | 1 | H | H | $CH_3$ | H | 0 | $OC_2H_5$ | mp.: 43° C *) |
| 105 | 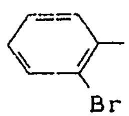 | 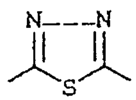 | O | 1 | H | H | $CH_3$ | H | 0 | $OC_2H_5$ | $n_D^{20}$ : 1,5831*) |
| 106 | 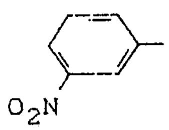 | 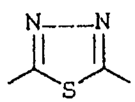 | O | 1 | H | H | $CH_3$ | H | 0 | $OC_2H_5$ | mp.: 65° C *) |
| 107 | 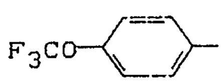 | 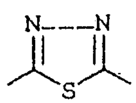 | O | 1 | H | H | $CH_3$ | H | 0 | $OC_2H_5$ | (Öl) *) |
| 108 | 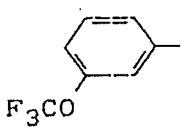 | 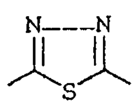 | O | 1 | H | H | $CH_3$ | H | 0 | $OC_2H_5$ | $n_D^{20}$ : 1,5364*) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 109 | 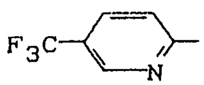 | 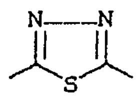 | O | 1 | H | H | $CH_3$ | H | O | $OC_2H_5$ | mp.: 42° C *) |
| 110 | 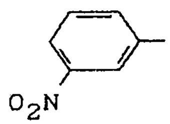 | 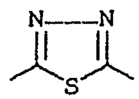 | O | 1 | H | H | $CH_3$ | H | O | $OC_2H_5$ | δ = 8,63; 8,32; 8,22; 7,69; 7,30; 6,94 *) |
| 111 | 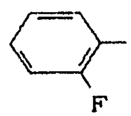 | 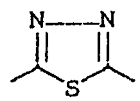 | O | 1 | H | H | $CH_3$ | H | O | $OCH(CH_3)CH_2OCH_2$– 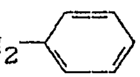 | (Öl) *) |
| 112 | 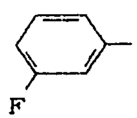 | 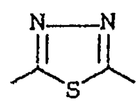 | O | 1 | H | H | $CH_3$ | H | O | $OCH_2CH_2OCH_2$– 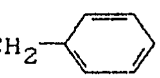 | δ = 4,78; 4,53; 4,38; 3,70; 1,64 *) |
| 113 | 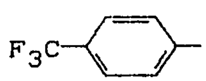 | 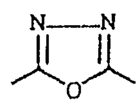 | O | 1 | H | H | $CH_3$ | H | O | $OC_2H_5$ | mp.: 45° C *) |
| 114 | 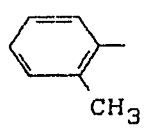 | 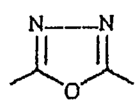 | O | 1 | H | H | $CH_3$ | H | O | $OC_2H_5$ | mp.: 52° C *) |

Tabelle 2 - Fortsetzung

| Bsp.-Nr. | Ar | Q | A | n | $X^1$ | $X^2$ | $R^1$ | $R^2$ | m | Y | physikalische Daten |
|---|---|---|---|---|---|---|---|---|---|---|---|
| 115 | 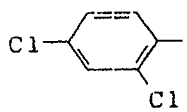 | 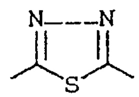 | O | 1 | H | H | $CH_3$ | H | O | OH | (amorph) *) |
| 116 | 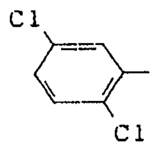 | 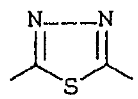 | O | 1 | H | H | $CH_3$ | H | O | OH | (amorph) *) |
| 117 | 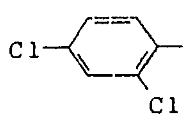 | 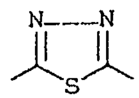 | O | 1 | H | H | $CH_3$ | H | O | $OC_2H_5$ | (amorph) *) |
| 118 | 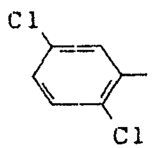 | 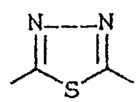 | O | 1 | H | H | $CH_3$ | H | O | $OC_2H_5$ | (amorph) *) |
| 119 | 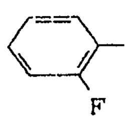 | 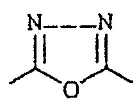 | O | 1 | H | H | $CH_3$ | H | O | $OC_2H_5$ | $n_D^{20}$ = 1,5553*) |

*) An dem mit * markierten C-Atom R-konfiguriert (vgl. Beispiel 2); die δ-Werte ergeben sich durch $^1$H-NMR-Messungen bei 300 MHz in $CDCl_3$; mp steht für Schmelzpunkt.

Ausgangsstoffe der Formel (II):

Beispiel (II-1)

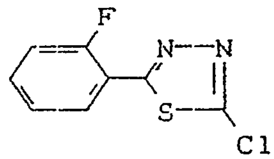

Stufe 1:

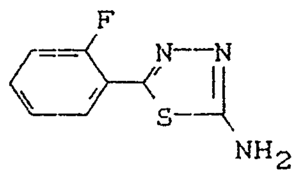

Zu einer durch portionsweises Eintragen von 30,4 g (1/3 mol) Thiosemicarbazid in 175 g 70%ige Schwefelsäure vorbereiteten Reaktionslösung gibt man bei einer Temperatur von 50-60° C 40,4 g (1/3 mol) 2-Fluorbenzonitril. Die Zutropfgeschwindigkeit des Nitrils wird dabei so gewählt, daß die Innentemperatur nicht über den genannten Bereich hinaus ansteigt. Man rührt 14h bei 55-60° C nach, gießt den Ansatz nach dem Abkühlen auf 2000 ml Eiswasser und neutralisiert durch Einrühren von Natronlauge. Der abgeschiedene Feststoff wird abgesaugt, nacheinander mit Wasser, Petrolether und wenig Ether gewaschen und getrocknet.

Man erhält 42,8 g (66 %) 2-Amino-5-(2-fluorphenyl)-1,3,4-thiadiazol vom Schmelzpunkt > 210° C (Zersetzung).

Stufe 2:

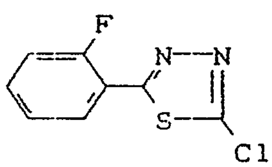

19,5 g (0,1 mol) 2-Amino-5-(2-fluorphenyl)-1,3,4-thiadiazol werden bei -10° C zusammen mit 3,2 g Kupferpulver und 0,1 g Kupfer(I)chlorid in 250 ml konzentrierter Salzsäure vorgelegt. Bei dieser Temperatur tropft man innerhalb von 30 Minuten eine Lösung von 13,8 g (0,2 mol) Natriumnitrit in 40 ml Wasser zu. Nach beendeter Zugabe rührt man 2 Stunden bei 0° C und weitere 2 Stunden bei 50° C nach und gibt den Ansatz dann auf 3000 ml Eiswasser. Das ausfallende Produkt wird abgesaugt, mit Wasser neutral gewaschen und getrocknet.

Man erhält 18,6 g (87%) gelbes 2-Chlor-5-(2-fluorphenyl)-1,3,4-thiadiazol vom Schmelzpunkt 103° C.

Beispiel (II-2)

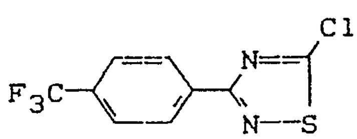

Zu 11,2 g (0,05 mol) 4-Trifluormethylbenzamidinhydrochlorid in 100 ml Dichlormethan gibt man bei 0° C innerhalb von 30 Minuten 9,3 g (0,05 mol) Perchlormethylmercaptan. Man rührt 30 Minuten bei 0° C nach, gibt dann innerhalb von 60 Minuten eine Lösung von 6,0 g (0,15 mol) Natriumhydroxid in 20 ml Wasser hinzu und rührt weitere 90 Minuten unter Eiskühlung. Danach wird die organische Phase abgetrennt, mit Wasser gewaschen, getrocknet und im Vakuum eingeengt.

Man erhält 4,5 g (34%) 3-(4-Trifluormethylphenyl)-5-chlor-1,2,4-thiadiazol als gelben Feststoff.

$^1$H-NMR (300 MHz, DMSO-$d_6$): $\delta$ = 7,94 und 8,37 (AA'BB'-System).

Beispiel (II-3)

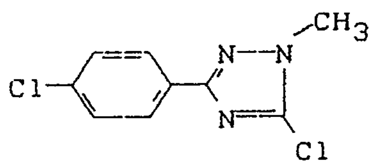

21 g (0,1 mol) 2-Methyl-5-(4-chlorphenyl)-1,2,4-triazolin-3-on werden in 150 ml Phosphoroxychlorid unter Zusatz einer katalytischen Menge Triphenylphosphinoxid 7 Stunden unter Rückfluß gekocht. Den Überschuß an Phosphoroxychlorid destilliert man unter vermindertem Druck ab und rührt den flüssigen Rückstand in 300 ml Eiswasser ein. Der Niederschlag wird abgesaugt, mit Eiswasser gewaschen und im Exsikkator getrocknet.

Man erhält 15,1 g (66% der Theorie) 2-Methyl-3-chlor-5-(4-chlorphenyl)-1,2,4-triazol als farblose Kristalle vom Schmelzpunkt 153-155° C.

Analog zu den Beispielen (II-1) bis (II-3) können beispielsweise auch die in der nachstehenden Tabelle 3 aufgeführten Verbindungen der Formel (II) hergestellt werden.

Ar-Q-$X^3$ (11)

Tabelle 3: Beispiele für die Ausgangsstoffe der Formel (II)

| Bsp.-Nr. | Ar | Q | $X^3$ | physikalische Daten |
|---|---|---|---|---|
| II-4 | 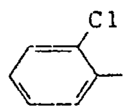 | 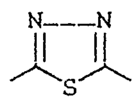 | Cl | mp.:82° C |
| II-5 | 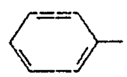 | 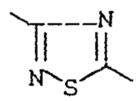 | Cl | mp.:40° C |
| II-6 | 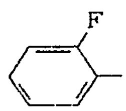 | 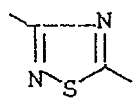 | Cl | δ = 7,1-7,3, 7,4-7,55, 8,1-8,2 |
| II-7 | 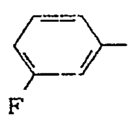 | 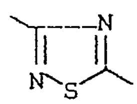 | Cl | δ = 7,19, 7,46, 7,94, 8,04 |
| II-8 | 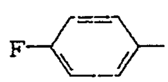 | 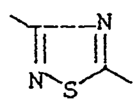 | Cl | δ = 7,16, 8,25 |
| II-9 | 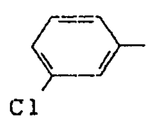 | 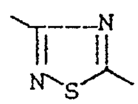 | Cl | δ = 7,35-7,50, 8,13, 8,25 |
| II-10 | 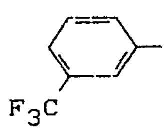 | 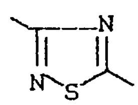 | Cl | δ = 7,61, 7,75, 8,43, 8,53 |

Die δ-Werte ergeben sich durch $^{1}$H-NMR-Messung bei 300 MHz in $CDCl_3$.

Ausgangsstoffe der Formel (IV):

Beispiel (IV-1)

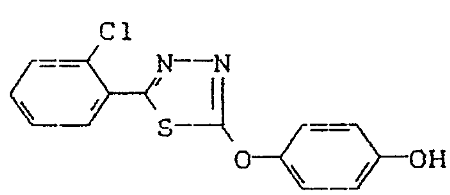

Eine Mischung aus 26,4 g (0,24 mol) Hydrochinon, 100 ml Dimethylsulfoxid und 7,6 g Kaliumhydroxid-Pulver (88%ig, 0,12 mol KOH) wird 30 Minuten bei 50° C gerührt. Dann wird eine Lösung von 23,1 g (0,10 mol) 2-Chlor-5-(2-chlorphenyl)-1,3,4-thiadiazol in 250 ml Dimethylsulfoxid innerhalb von 2 Stunden zugetropft und das Reaktionsgemisch 5 Stunden bei 50° C gerührt. Dann wird mit Wasser auf etwa das vierfache Volumen verdünnt, mit 2N-Salzsäure auf pH = 2 angesäuert und das kristalline Produkt durch Absaugen isoliert. Das Produkt wird zur weiteren Reinigung in Wasser suspendiert, durch Zugabe von 2N-Natronlauge in der Hitze nahezu in Lösung gebracht und filtriert. Das Filtrat wird durch Zugabe von konzentrierter Salzsäure auf pH = 1 gestellt und das hierbei kristallin anfallende Produkt durch Absaugen isoliert.

Man erhält 18,9 g (62% der Theorie) 2-(4-Hydroxy-phenoxy)-5-(2-chlor-phenyl)-1,3,4-thiadiazol.

Beispiel (IV-2)

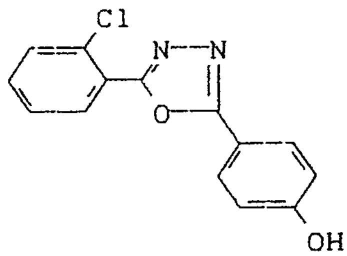

Ein Gemisch aus 7,8 g (0,05 mol) 2-Chlorbenzoesäure, 7,6 g (0,05 mol) 4-Hydroxybenzhydrazid und 11,5 g (0,075 mol) Phosphoroxychlorid wird 5 Stunden auf 106° C erhitzt. Das erkaltete Reaktionsprodukt wird gemörsert, mit Wasser gewaschen und aus Isopropanol umkristallisiert.

Man erhält 6,5 g eines farblosen Feststoffs, der nach einer GCMS-Untersuchung (m/z = 272, M+, 100% rel. Int.) zu etwa 35% aus 2-(2-Chlorphenyl)-5-(4-hydroxyphenyl)-1,3,4-oxadiazol besteht. Dieses Zwischenprodukt wird ohne weitere Reinigung gemäß Verfahren (b) zu entsprechenden Verbindungen der Formel (I) weiter umgesetzt.

Analog zu den Beispielen (IV-1) und (IV-2) können beispielsweise auch die in der nachstehenden Tabelle 4 aufgeführten Verbindungen der Formel (IV) hergestellt werden.

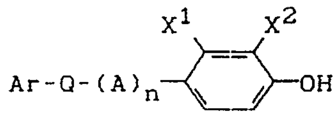

(IV)

Tabelle 4: Beispiele für die Verbindungen der Formel (IV)

| Bsp.-Nr. | Ar | Q | A | n | $X^1$ | $X^2$ | Schmelzpunkt (°C) |
|---|---|---|---|---|---|---|---|
| IV-3 | 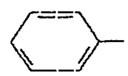 | 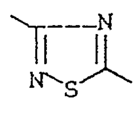 | O | 1 | H | H | glasartig |
| IV-4 | 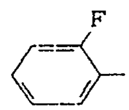 | 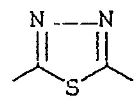 | O | 1 | H | H | 145 |
| IV-5 | 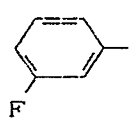 | 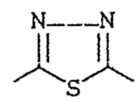 | O | 1 | H | H | 120 |

Anwendungsbeispiele:

In den nachstehend beschriebenen Anwendungsbeispielen wurde die folgende Verbindung als Vergleichssubstanz eingesetzt:

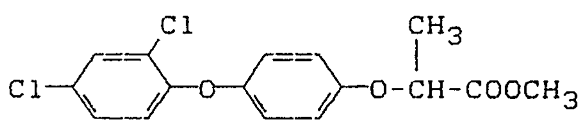

(A)

2-[4-(2,4-Dichlorphenoxy)-phenoxy]-propionsäuremethylester (bekannt aus DE-OS 22 23 894)

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen (1), (13) und

(14) bei guter Verträglichkeit gegenüber Nutzpflanzen, wie z.B. Weizen, Gerste, Raps und Soja, eine bis zu 100 % stärkere Wirkung gegen Unkräuter als die bekannte Verbindung (A).

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen (1) und (20) bei guter Verträglichkeit gegenüber Nutzpflanzen, wie z.B. Reis, starke Wirkung gegen Unkräuter.

**Patentansprüche**

**1.** Aryloxycarbonylverbindungen der allgemeinen Formel (I)

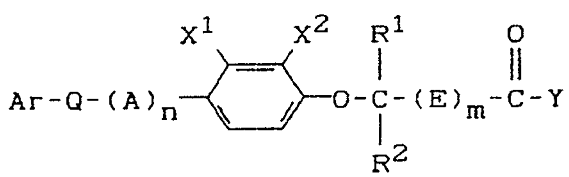

(I)

in welcher

| | |
|---|---|
| m und n | für die Zahlen 0 oder 1 stehen, |
| Ar | für Aryl oder Heteroaryl steht, |
| Q | für Heteroarylen steht, |
| A | für Sauerstoff, Schwefel oder Methylen steht, |
| $X^1$ und $X^2$ | gleich oder verschieden sind und für Wasserstoff, Halogen, Alkyl, Halogenalkyl, Alkoxy oder Halogenalkoxy stehen, |
| $R^1$ und $R^2$ | gleich oder verschieden sind und für Wasserstoff, Alkyl oder Halogenalkyl stehen, |
| E | für eine gegebenenfalls substituierte Ethylen- oder Vinylen-Gruppe steht, |
| | und |
| Y | für den Rest einer Carbonsäure oder eines Carbonsäurederivats steht. |

**2.** Verbindungen der Formel (I), gemäß Anspruch 1,

in welcher

| | |
|---|---|
| m und n | für die Zahlen 0 oder 1 stehen, |
| Ar | für Phenyl oder Pyridyl steht, welche jeweils gegebenenfalls durch Halogen, Cyano, Nitro und/oder durch jeweils gegebenenfalls halogensubstituiertes Alkyl, Alkoxy, Alkylthio, Alkylsulfinyl oder Alkylsulfonyl mit jeweils 1 bis 6 Kohlenstoffatomen im Alkylteil substituiert sind, |
| Q | für einen fünf- oder sechsgliedrigen Heteroarylenrest steht, welcher 2 cder 3 Stickstoffatome (von denen gegebenenfalls eines alkyiert ist) oder 1 oder 2 Stickstoffatome und zusätzlich 1 Sauerstoffatom oder Schwefelatom als Heteroatome enthält, steht, |

A für Sauerstoff, Schwefel oder Methylen steht,

$X^1$ und $X^2$ gleich oder verschieden sind und für Wasserstoff, Halogen, Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen stehen,

$R^1$ und $R^2$ gleich oder verschieden sind und für Wasserstoff, Alkyl oder Halogenalkyl mit jeweils 1 bis 4 Kohlenstoffatomen und 1 bis 9 gleichen oder verschiedenen Halogenatomen stehen,

E für die Gruppierungen -CH=CH- oder

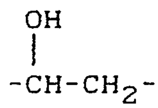

steht und

Y für Halogen, Hydroxy, Amino, Hydrazino, Cyano, Cyanamino, Hydroxyamino, Isoxazolidinyl oder für jeweils gegebenenfalls durch Halogen substituiertes $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Di-($C_1$-$C_4$-alkyl)-amino, Pyrrolidino, Piperidino, Morpholino, Di-($C_3$-$C_4$-alkenyl)amino, $C_1$-$C_4$-Alkylsulfonylamino, Phenylsulfonylamino, Tolylsulfonylamino, $C_1$-$C_6$-Alkoxyamino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^3$ steht, worin

$R^3$ für jeweils gegebenenfalls durch Fluor und/oder Chlor substituiertes $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkoxy-$C_1$-$C_4$-alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino, oder für ein Ammonium-, ein $C_1$-$C_4$-Alkylammonium-, ein Natrium-, Kalium- oder Calciumäquivalent steht, oder

$R^3$ für die Gruppierung

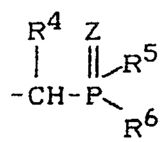

steht, worin

$R^4$ für Wasserstoff, $C_1$-$C_4$-Alkyl, Phenyl, Furyl, Thienyl oder Pyridyl steht,

$R^5$ für $C_1$-$C_4$-Alkyl oder $C_1$-$C_4$-Alkoxy steht,

$R^6$ für $C_1$-$C_4$-Alkoxy steht und

Z für Sauerstoff oder Schwefel steht,

oder

$R^3$ für die Gruppierung -$(CH_2)_p$-$R^7$ steht, worin

$R^7$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Isoxazolyl, Thiazolyl, Thiadiazolyl, Dioxolanyl, Perhydropyrrolyl, Oxoperhydropyrrolyl, Pyridinyl, Pyrazolyl oder Pyrimidinyl steht und

p für die Zahlen 0, 1 oder 2 steht.

**3.** Verbindungen der Formel (I), gemäß Anspruch 1,

in welcher

m und n — für die Zahlen 0 oder 1 stehen,

Ar — für Phenyl oder Pyridyl steht, welche jeweils gegebenenfalls durch Fluor, Chlor, Brom, Cyano, Nitro und/oder durch jeweils gegebenenfalls durch Fluor und/oder Chlor substituiertes $C_1$-$C_4$-Alkyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert sind,

Q — für Pyrazin-2,6-diyl, Pyrazin-2,5-diyl, 1,2,4-Triazin-3,5-diyl, 1,2,4-Triazin-3,6-diyl, 1,3-Imidazol-2,5-diyl, 1,3-Imidazol-2,4-diyl oder 1,2,4-Triazol-3,5-diyl (welche jeweils gegebenenfalls durch $C_1$-$C_4$-Alkyl substituiert sind), für 1,3,4-Oxadiazol-2,5-diyl, 1,3,4-Thiadiazol-2,5-diyl, 1,2,4-Oxadiazol-3,5-diyl, 1,2,4-Thiadiazol-3,5-diyl, 1,3-Oxazol-2,5-diyl, 1,3-Thiazol-2,5-diyl, 1,3-Oxazol-2,4-diyl oder 1,3-Thiazol-2,4-diyl steht,

A — für Sauerstoff, Schwefel oder Methylen steht,

$X^1$ und $X^2$ — gleich oder verschieden sind und für Wasserstoff, Fluor, Chlor oder Halogenalkyl mit 1 oder 2 Kohlenstoffatomen und 1 bis 5 gleichen oder verschiedenen Halogenatomen stehen,

$R^1$ und $R^2$ — gleich oder verschieden sind und für Wasserstoff, $C_1$-$C_4$-Alkyl oder Halogenalkyl mit 1 bis 4 Kohlenstoffatomen und 1 bis 9 gleichen oder verschiedenen Halogenatomen stehen,

E — für die Gruppierungen -CH = CH- oder

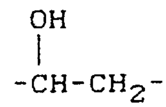

steht

und

Y — für Chlor, Hydroxy, Amino, Hydrazino, Cyano, Cyanamino, Hydroxyamino, Isoxazolidinyl oder für jeweils gegebenenfalls durch Fluor und/oder Chlor substituiertes $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Di-($C_1$-$C_4$-alkyl)-amino, Pyrrolidino, Piperidino, Morpholino, Di-($C_3$-$C_4$-alkenyl)-amino, $C_1$-$C_4$-Alkylsulfonylamino, Phenylsulfonylamino, Tolylsulfonylamino, $C_1$-$C_6$-Alkoxyamino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^3$ steht, worin

$R^3$ — für jeweils gegebenenfalls durch Fluor und/ oder Chlor substituiertes $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkoxy-$C_1$-$C_4$-alkoxy-$C_1$-$C_3$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Pyrazolyl-$C_1$-$C_4$-alkyl, $C_2$-$C_4$-Alkylidenamino, oder für ein Ammonium-, ein $C_1$-$C_4$-Alkylammonium-, ein Natrium-, Kalium- oder Calciumäquivalent steht, oder

$R^3$ — für die Gruppierung

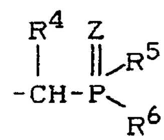

steht, worin

$R^4$ — für Wasserstoff, $C_1$-$C_4$-Alkyl, Phenyl, Furyl, Thienyl oder Pyridyl steht,

$R^5$ — für $C_1$-$C_4$-Alkyl oder $C_1$-$C_4$-Alkoxy steht,

$R^6$ — für $C_1$-$C_4$-Alkoxy steht und

Z — für Sauerstoff oder Schwefel steht,

oder

$R^3$ für die Gruppierung $-(CH_2)_p-R^7$ steht, worin

$R^7$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Isoxazolyl, Thiazolyl, Thiadiazolyl, Dioxolanyl, Perhydropyrrolyl, Oxoperhydropyrrolyl, Pyridinyl, Pyrazolyl oder Pyrimidinyl steht und

p für die Zahlen 0, 1 oder 2 steht.

**4.** Verbindungen der Formel (I), gemäß Anspruch 1,

in welcher

m für die Zahl 0 steht,

n für die Zahl 0 oder 1 steht,

Ar für Phenyl oder Pyridyl steht, welche gegebenenfalls einfach oder zweifach durch Fluor und/oder Chlor und/oder einfach durch Methyl, Trifluormethyl, Methoxy, Difluormethoxy, Trifluormethoxy, Methylthio oder Trifluormethylthio substituiert sind,

Q für 1,3,4-Oxadiazol-2,5-diyl, 1,3,4-Thiadiazol-2,5-diyl, 1,2,4-Oxadiazol-3,5-diyl, 1,2,4-Thiadiazol-3,5-diyl, 1,2,4-Triazol-3,5-diyl, 1-Methyl-1,2,4-triazol-3,5-diyl, 4-Methyl-1,2,4-triazol-3,5-diyl, 1,3-Oxazol-2,5-diyl, 1,3-Oxazol-2,4-diyl, 1,3-Thiazol-2,5-diyl oder 1,3-Thiazol-2,4-diyl steht,

A für Sauerstoff steht,

$X^1$ und $x^2$ für Wasserstoff stehen,

$R^1$ für Methyl oder Wasserstoff steht,

$R^2$ für Wasserstoff steht und

Y für Chlor, Hydroxy, Amino, Isoxazolidinyl oder für jeweils gegebenenfalls durch F- und/oder Cl-substituiertes $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, $C_1$-$C_4$-Alkyl-sulfonylamino, Phenylsulfonylamino, $C_1$-$C_4$-Alkoxyamino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung $-O-R^3$ steht, worin

$R^3$ für jeweils gegebenenfalls durch Cl- und/oder F-substituiertes $C_1$-$C_4$-Alkyl, $C_1$-$C_2$-Alkoxy-$C_1$-$C_2$-alkyl, $C_1$-$C_2$-Alkoxy-$C_1$-$C_2$-alkoxy-methyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl, Propargyl oder für ein Ammonium-, $C_1$-$C_3$-Alkylammonium-, Natrium-, oder Kaliumäquivalent steht, oder

$R^3$ für die Gruppierung $-(CH_2)_p-R^7$ steht, worin

$R^7$ für einen heterocyclischen Rest aus der Reihe Tetrahydrofuryl, Isoxazolyl oder Pyrazolyl steht und

p für die Zahlen 1 oder 2 steht.

**5.** Verfahren zur Herstellung von neuen Aryloxycarbonylverbindungen der Formel (I)

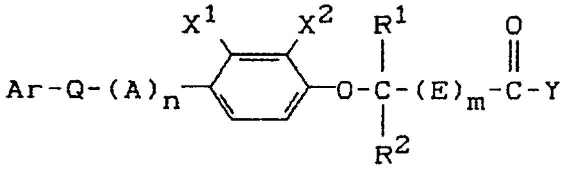

(I)

in welcher

m und n für die Zahlen 0 oder 1 stehen,

Ar für Aryl oder Heteroaryl steht,

Q für Heteroarylen steht,

A für Sauerstoff, Schwefel oder Methylen steht,

$X^1$ und $X^2$ gleich oder verschieden sind und für Wasserstoff, Halogen, Alkyl, Halogenalkyl, Alkoxy

oder Halogenalkoxy stehen,

$R^1$ und $R^2$ gleich oder verschieden sind und für Wasserstoff, Alkyl oder Halogenalkyl stehen,

E für eine gegebenenfalls substituierte Ethylen- oder Vinylen-Gruppe steht,

und

Y für den Rest einer Carbonsäure oder eines Carbonsäurederivats steht, dadurch gekennzeichnet, daß man

(a) Arylverbindungen der allgemeinen Formel (II)

$Ar\text{-}Q\text{-}X^3$ (11)

in welcher

Ar und Q die oben angegebenen Bedeutungen haben und

$X^3$ für eine nucleofuge Gruppe steht,

mit Hydroxy- oder Mercaptoaryloxycarbonylverbindungen der allgemeinen Formel (III)

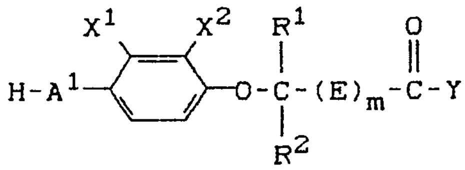

(III)

in welcher

m, $X^1$, $X^2$, $R^1$, $R^2$, E und Y die oben angegebenen Bedeutungen haben und

$A^1$ für Sauerstoff oder Schwefel steht,

gegebenenfalls in Gegenwart eines Säureakzeptors und gegebenenfalls in Gegenwart eines Verdünnungsmittels umsetzt, oder wenn man

(b) Phenolderivate der allgemeinen Formel (IV)

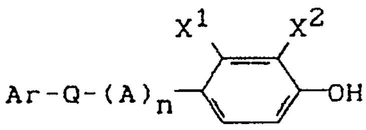

(IV)

in welcher

n, Ar, Q, A, $X^1$ und $X^2$ die oben angegebenen Bedeutungen haben,

mit Carbonsäurederivaten der allgemeinen Formel (V)

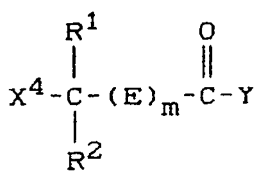

(V)

in welcher

m, $R^1$, $R^2$, E und Y die oben angegebenen Bedeutungen haben und

$X^4$ für eine nucleofuge Gruppe steht,

gegebenenfalls in Gegenwart eines Säureakzeptors und gegebenenfalls in Gegenwart eines Verdünnungsmittels umsetzt.

6. Herbizide Mittel, gekennzeichnet durch einen Gehalt an mindestens einer Aryloxycarbonylverbindung der Formel (I) nach den Ansprüchen 1 bis 5.

7. Verwendung von Aryloxycarbonylverbindungen der Formel (I) nach den Ansprüchen 1 bis 5 zur Bekämpfung von unerwünschten Pflanzen.

8. Verfahren zur Bekämpfung von unerwünschten Pflanzen dadurch gekennzeichnet, daß man Aryloxycarbonylverbindungen der Formel (I) nach den Ansprüchen 1 bis 5 auf Pflanzen und/oder ihren Lebensraum einwirken läßt.

9. Verfahren zur Herstellung von herbiziden Mitteln, dadurch gekennzeichnet, daß man Aryloxycarbonylverbindungen der Formel (I) nach den Ansprüchen 1 bis 5 mit Streckmitteln und/oder oberflächenaktiven Mitteln vermischt.

10. Verbindungen der Formel (IV)

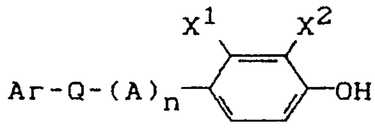

(IV)

in welcher

n, Ar, Q, A, $X^1$ und $x^2$ die in Anspruch 1 angegebene Bedeutung haben.

11. Verfahren zur Herstellung von Verbindungen der Formel (IV)

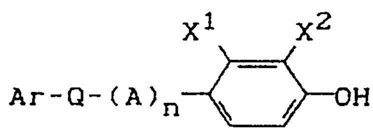

(IV)

in welcher

n, Ar, Q, A, $X^1$ und $X^2$ die in Anspruch 1 angegebene Bedeutung haben,

dadurch gekennzeichnet, daß man

a) für den Fall, daß in Formel (IV) n für die Zahl 1 und A für Sauerstoff oder Schwefel steht, Arylverbindungen der Formel (II)

$Ar - Q - X^3$ (II)

in welcher

Ar und Q die in Anspruch 1 angegebenen Bedeutungen haben und $X^3$ für eine nucleofuge Gruppe steht, mit Phenolen der Formel (VI)

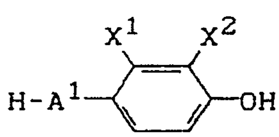

(VI)

in welcher

$X^1$ und $X^2$ die in Anspruch 1 angegebenen Bedeutungen haben und $A^1$ für Sauerstoff oder Schwefel steht,

in Gegenwart eines Säureakzeptors und in Gegenwart eines Verdünnungsmittels bei Temperaturen

zwischen 0 °C und 150 °C umsetzt,

oder

b) für den Fall, daß in Formel (IV) n für die Zahl 1 und A für Methylen oder n für die Zahl 0 steht, Arylverbindungen der Formel (VII)

Ar - $Q^1$ (VII)

in welcher Ar die in Anspruch 1 angegebene Bedeutung hat und $Q^1$ für einen organischen Rest mit ein oder zwei Kohlenstoffatomen steht,

mit Phenolen der Formel (VIII)

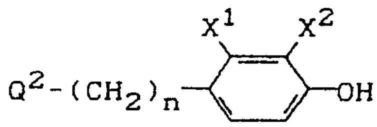

(VIII)

in welcher n, $X^1$ und $X^2$ die in Anspruch 1 angegebenen Bedeutungen haben und $Q^2$ für einen organischen Rest mit ein oder zwei Kohlenstoffatomen steht, umsetzt,